# EUROPEAN PATENT SPECIFICATION

(11) **EP 1 842 568 B1**
(45) Date of publication and mention of the grant of the patent: **11.06.2008**
(21) Application number: 06425234.9
(22) Date of filing: 05.04.2006
(51) Int. Cl.: A61L 31/04, A61L 31/14

(54) **Multimicrolamellar collagen membranes**
Multimikrolamellare Kollagenmembranen
Membranes de collagène multimicrolaminaires

(43) Date of publication of application: 10.10.2007
(73) Proprietor: OPOCRIN S.p.A., 41040 Corlo di Formigine (Modena) (IT)
(72) Inventor: Parma, Bruna, 41100 Modena (IT); Gigante, Antonio, 60129 Ancona (IT)
(74) Representative: Gervasi, Gemma

(56) References cited:
- EP-A- 1 307 247
- WO-A-95/18638

## Description

Collagen is a scleroprotein with mechanical functions, that is mostly present in the animal and human connective tissue. In human, collagen makes about 30% of total proteins and is the primary component of cartilage, ligaments and tendons.

Several types of collagen exist that are mainly differentiated in relation to the function of the tissue from which they originate. It is well known that the properties of collagen depend not only on the structure of protein monomers composing the triple helix of the collagen fiber, but mainly on the complex macromolecular organization that distinguishes this and other structural proteins with a mechanical function (e.g. myosin and tropomyosin in muscle fibers, etc) and which involves an organization in fibrils, their supercoiling, their head-to-tail assembly and so on.

The molecular organization of native collagen fibers is partially lost during collagen extraction procedures. It can be partly restored artificially, for instance by chemical cross-linking. This treatment restores, even though only partially, the mechanical resistance of collagen fibrils and also makes the final product more resistant to proteolytic digestion.

So far, several collagen-based preparations have been introduced in medical practice, including preparations in which collagen is present together with other natural components, either in the form of gel and felt, membrane or patch, as stimulating agents for wound cicatrization, or as vehicle or device for the slow release of other drugs or as matrices suitable for interactions with cells. In fact, the importance of collagen as substrate that favours cellular interactions and growth is well known. It is also well known that cell lines of different origin exhibit preferences for different types of collagen: for instance skin fibroblasts and epithelial cells (Murray J.C. et al. Cancer Res. 40,347, 1980; Wicha M.S. et al. Exp. Cell Res. 124,81, 1979) grow better on type IV collagen (the collagen present in the basal membrane) whereas chondrocytes prefer type II collagen (that is present in hyaline cartilage) (Hewit A.T. et al. Proc. Natl. Acad. Sci. USA 77,385, 1980) and adapt well also to grow on type I and II collagens.

The presence of fibronectin, endogenously produced by cells (Grinnel F. et al. Proc. Natl. Acad. Sci. USA 75,4408(1978) has been proven to be important in this type of application mediating fibroblast adhesion to the collagen substrate (Pearlstein E., Int. J. Cancer 22,32, 1978), and of chondronectin, mediating the interaction of chondrocytes with collagen (Hewit et al. Proc. Natl. Acad. Sci. USA 77,385, 1980).

Methods for extraction and preparation of collagen membranes are described in US 5,785,983 claiming a process for preparation of collagen membranes composed by chemically unmodified type I collagen, that is obtained from collagen gels, placed in non-adherent Teflon trays, by slow drying, under suitable conditions of temperature, vacuum and nitrogen flow. The preparation of collagen gels from bovine tendon is also disclosed in WO98/44809.

Methods for preparation of felts or collagen membranes by freezing and quick drying of collagen solutions, in the form of gels, have also been described. The structure of said felts is not organized, as such procedures fix as in an instantaneous photograph the disorder of collagen fibers in solution. The resistance of these membranes is limited because the collagen, that is denatured in the extraction process, is not sufficiently reorganized.

Patent WO95/18638 claims reabsorbable collagen membranes as a guide in tissue repair, characterized by two opposite sides, of which one is fibrous and suitable for cell growth and the other is smooth and suitable to prevent adherences. Said membranes are obtained from peritoneal or placental membranes of several mammals, including calf, prepared by a gross process of cleaning, dehydration and degreasing with acetone and N-hexane. Due to the preparation method employed, membranes have the same size as the organ from which they originate, and also their peculiar features, such as porosity and type of collagen, reflect those of the tissue of origin.

Patent WO 96/25961 claims reabsorbable matrices of type II collagen (from cartilage) suitable for the reconstruction of natural cartilagineous tissues. Said matrices are prepared by freezing and lyophilization and can be integrated with glycosaminoglycans.

Patent WO 99/19005 claims a multilayered membrane comprising of a matrix primarily made of type II collagen, composed of a spongious side and a relatively impermeable side. Said membrane is prepared by sequential stratification of collagen matrices (previously degreased with acetone); during subsequent, discontinuous operations, portions of collagen gel are then lyophilized on such membranes.

Thus, up to now, collagen membrane preparation procedures involve either the extraction of collagen in a solid, semi-purified form or its solubilization in the form of gel that is then rapidly frozen and desiccated. After desiccation, collagen is generally also chemically modified by cross-linking that occurs randomly and prevents the macromolecular reorganization typical of the native fibril. Alternatively, membranes can be prepared by slow evaporation of a collagen gel or they can be prepared directly from naturally pre-existing collagen membranes that are only partially purified.

Patent EP1307247 B1 describes the preparation of collagen membranes from collagen, by exsiccation, in a magnetic field. During exsiccation, membranes acquire a macromolecular organization that can be shown by scanning electron microscopy on one side of the membrane, a feature that makes them especially suitable as coadjuvant support for cell growth.

The process described in EP1307247 B1 is further optimized to generate membranes endowed with complex molecular microstructures with distinct and original characteristics compared to those already described.

### SUMMARY OF THE INVENTION

The present invention relates to multimicrolamellar collagen membranes of several types and structures (e.g. type I, type II, type III, etc.), of only one type or two types in association, but more particularly of type I and type II and/or of their mixture.

The multimolecular arrangement is obtained by building the membrane in several steps involving the sequential addition of collagen gel.

Membranes look thin, with a variably rough surface depending on the type of collagen used but always with a parallel horizontal lamellar microstructure so that they can be soaked with different fluids, act as three-dimensional scaffold for cultured cells and are apt to be penetrated and colonized by cells in vivo, therefore functioning as a support suitable for direct, guided tissue regeneration.

Furthermore, the present invention relates to the preparation of said membranes from collagen gels and to optimization of the preparation of the collagen gel from tissues.

### SHORT DESCRIPTION OF THE FIGURES

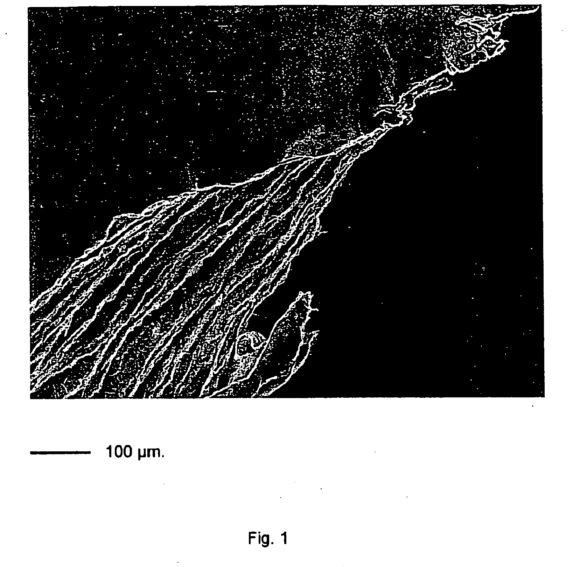

**Figure 1****. Analysis by scanning electron microscope (SEM).** SEM image of collagen I membrane lot 40126 at 250x magnification. The presence of the parallel microlamellae is evident.
**Figure 2****. Histogram.** This histogram illustrates the resistance to traction of membranes with identical thickness and composed by the same amount of collagen (9 mg/cm²), however prepared either by the collagen multistratification process (multimicrolamellar 40126) or according to the prior art (40124): the multimicrolamellar membrane 40126 is much more resistant to traction than 40124.
**Figure 3****. Analysis by scanning electron microscope.** Scanning electron microscope (SEM) analysis reveals the multimicrolamellar structure of membrane 030718 made of type II collagen from equine joint cartilage. 150x magnification.
**Figure 4****. Histogram**. This histogram illustrates the thickness of joint cartilages in the site of damage after 6 months of the treatment with type I collagen membranes prepared according to EP1307247 (40124), multimicrolamellar type I collagen (40126), multimicrolamellar mixture of type I and II collagen (C2A40) and with type I and type II collagen as distinct layers (C1+C2).
**Figure 5****. Section of joint cartilage lesion - Control at 3 months.** The section stained with Safranin-O evidentiates the surface of the joint, which is empty or partially filled with fibrin clots, comes from control animals at 3 months. The lesion appears clearly delimited and morphologically distinct from the surrounding cartilage. 20x magnification.
**Figure 6****. Section of joint cartilage lesion - Control at 9 months.** This section has been taken from control animals after 9 months and was stained with Safranin-O. The staining reveals the disappearance of the columnar arrangement of the cellular component, notches of the more superficial layers, alteration of the characteristics colours of the matrix, chondrocyte pycnosis. 100x magnification.
**Figure 7****. Section of joint cartilage lesion- Treated at 9 months.** The lesion has been treated with membrane I/II and was taken after 9 months. Safranin-O staining reveals a repair tissue that is well anchored to the underlying bone, composed of cartilage cells and a hyaline-type cartilaginous matrix. It is possible to observe a tangential arrangement of the most superficial cells and, in the deepest portion of the repair tissue, a normal osteocartilaginous junction with formation of a *tide-mark.* 100x magnification.
**Figure 8****. Section of joint cartilage lesion - Treated at 9 months.** Lesion treated with membrane I/II after 9 months. Safranin-O stained section evidentiates the relation of the newly formed cartilage with the surrounding tissues. 20x magnification.
**Figure 9****. Section of joint cartilage lesion - Control at 12 months.** Control lesion after 12 months. Safranin-O stained section evidentiates the cartilaginous lesion, wherein only the lesion sharply distinct from the surrounding tissues is clear. 20x magnification.
**Figure 10****. Section of joint cartilage lesion.** Lesion treated with membrane known in the art, after 12 months. Safranin-O stained section evidentiates the lesion, in which only the fibrous repair tissue can be seen. 20x magnification.
**Figure 11****. Analysis by scanning electron microscope (SEM).**
   a) 100% type I collagen multimicrolamellar membrane. Scanning electron microscopy (SEM) photograph. 2500x magnification.
   b) 100% type II collagen multimicrolamellar membrane. Scanning electron microscopy (SEM) photograph. 5520x magnification.
   c) Mixed type I and II collagen (60%-40%) multimicrolamellar membrane. Scanning electron microscopy (SEM) photograph. 5030x magnification.

### DETAILED DESCRIPTION OF THE INVENTION

The present invention relates to multilayered membranes made of collagen from various sources, in particular from equines or other mammals, particularly of type I and II collagen and/or of their mixture and arranged as overlapping, parallel microlamellae.

Due to the purification process applied to collagen, said membranes are biocompatible, non-cytotoxic, non-sensitising and non-immunogenic; furthermore, due to the production process used, they are reabsorbable, very hygroscopic and their thickness and collagen content can be modulated.

The arrangement as "overlapping and parallel microlamellae", hereafter also referred to as multilamellar, a definition that will be better explained below, is clearly distinct, by electron scanning microscopy (SEM), from that defined "macromolecular" arrangement, described in EP 1307247. Moreover, the multimicrolamellar structure is present throughout the membrane's depth, while the macromolecular structure is observed primarily at the surface level.

Membranes obtained by the procedure involving the sequential stratification of collagen gels, even if of different composition as further detailed below, have a collagen content that can be modulated depending on the number and composition of the gel layers poured. Generally this content is comprised from 6 to 60 mg/cm², preferably from 9 to 30 mg/cm² and preferably from 15 to 25 mg/cm². Within these limits, the yielding point value of the membranes is from - 0.005 to 50 Mpa (Megapascal). More particularly, the yielding point value is comprised from 0.01 to 0.1 Mpa, for membranes comprising exclusively or in part the type II collagen, and from 0.5 to 50 Mpa, or preferably from 2.5 to 15 Mpa, for membranes primarily composed of type I collagen.

The Young modulus (an index of mechanical resistance but also of rigidity of membranes) is from 0.001 to 100 Mpa. Young module and other properties of collagen membranes, such as deformability and yielding point, are measured by methods well known in the art, for instance as described in Falini G. et al . J Mater Chem, 2004, 14:2297-2302.

According to a preferred aspect, the membrane Young modulus is comprised from 0.01 to 0.1 Mpa, for membranes composed exclusively or in part by type II collagen, and preferably from 0.5 to 60 Mpa, and more preferably from 5 to 40 Mpa, for membranes essentially composed of type I collagen.

According to a preferred embodiment, the membranes of the invention are made of different types of collagen, preferably type I and II collagen, arranged either in different layers or in a way so that each layer contains different types of collagen. According to the latter embodiment, at least one layer of the membranes of the invention is composed by a mixture of type I and II collagen. Even more preferably said mixed composition a percentage of type II collagen that is from 10 to 70% (w/w) of the total collagen. Even more preferably, the percentage of type II collagen is comprised from 20 to 60% (w/w) or even more preferably the percentage is from 30 to 50% (w/w).

A further aspect of the present invention is a procedure for preparing collagen membranes, wherein collagen gel is poured into electrostatically charged trays or plates according to the known art and is then partially exsiccated and then under the same conditions, additional layers of collagen gel are poured on top of the previous layer.

The first layer is allowed to exsiccate until the residual water content is comprised between 30 and 70% or, according to a preferred embodiment, until the volume of the gel layer has been reduced of at least 30-70%. Said procedure is characterized in that the next layers of collagen gel are poured after partial exsiccation of the previous layer and the gel layer that was just poured is subjected to a vertical electrostatic field. The partial exsiccation of the first layer and of subsequent layers takes place preferably at a temperature comprised from 10 to 40°C, preferably from 20 to 30°C, even more preferably from 22 to 28°C, or even more preferably from 24 to 26°C, in an aerated incubator without turbulence, such as an incubator with diaphragmed air flow intake.

After partial exsiccation of the first layer, a volume of collagen gel is poured, whose composition may differ from the composition of the previous layer. After pouring, this gel layer, having a thickness from 1.5 to 20 mm, is optionally flattened with a spatula and then exsiccated under the same conditions described for the first layer.

Also the gel layer that was subsequently poured is exsiccated until the residual water content in the first or in the previous layer is from 30 to 70%, or, as seen above, until the original gel volume is reduced of at least 30-70%.

This procedure can be repeated until reaching the useful amount of collagen/ cm² or the desired thickness.

The trays for the collagen gels are exposed to a dispersion surface, such as water, in order to completely discharge their electrostatic charge, they are filled with gel, and then they are electrostatically charged by placing them for at least 2 minutes, and preferably 5 minutes, between two flat and parallel copper foils attached to a high voltage generator. The static electrical field with vertical orientation that develops between the two foils is at least 100 kv/m, more preferably comprised between 100-220 kv/m, between 150 and 200 kv/m, o even more preferably between 160 and 180 kv/m. Trays are then placed in the desiccator. At the moment when a second or a further load of collagen gel is added to the tray, the trays are transferred onto a dispersion surface, loaded with gel, placed in the vertical electrical field, then placed again in the exsiccator. Such a procedure is also applied to any subsequent load of collagen gel.

Therefore, schematically the procedure for preparation of membranes from collagen gels involves the following steps:
a) optional removal of electrostatic charges present on tray made of polymeric material, preferably polystyrene, by exposure to a dispersion surface or water;
b) loading of the tray in a static electrical field by interposition between two flat and parallel copper foils connected to a high voltage generator able to produce an electrostatic field with vertical orientation, of at least 100 kv/m, more preferably from 100 to 220 kv/m or from 150 to 200 kv/m, or more preferably from 160 to 180 kv/m, for at least 2' or preferably at least 5',
c) pouring of the first layer of collagen gel, until reaching a thickness from 1.5 to 20 mm, optionally flattening of the fluid layer with a spatula;
d) transfer to a static electrical field having characteristics identical to those in step b);
e) partial drying at constant temperature from 10 to 40°C, preferably between 20 and 30°C, even more preferably from 22 to 28°C, or even more preferably from 24 to 26°C, in an areated incubator without turbulence, such as an incubator with diaphragmed air flow intake, until the thickness of the gel is reduced by evaporation of 30-70% of the original volume, preferably of 40-60%, even more preferably of about 50% or until the residual water content is comprised between 70 and 30%;
f) repetition of steps a) and b) and pouring of the second or next layer of collagen gel, wherein said layer, still fluid, has a thickness comprised between 1.5 and 20 mm, as measured for instance with an Electronic outside micrometer IP54 IDF, and optional flattening of the fluid layer with a spatula;
g) repetition of steps d) and e), until partial exsiccation as in step e).

After pouring the last gel layer, the membrane is left under the same temperature conditions, but without ventilation, until complete or ≥ 80% exsiccation, resulting in the spontaneous detachment from the bottom of the template.

The membranes thus obtained can be sterilized, for instance by physical methods, such as γ-ray irradiation with a dose comprised between 2.5 and 25 Kgy.

Thus, in the process of the invention, the concomitant induction of an electrical field and of at least one additional pouring of collagen gel onto a first, partially exsiccated collagen layer, is associated with the observation, by electron microscopy, of a multimicrolamellar structure, wherein fibrils are arranged in an organized multimicrolamellar structure.

The gel used in the procedure according to the invention is made of collagen, in aqueous solution, at a concentration from 0.1 to 5% preferably from 0.4 3%, even more preferably from 0.8 to 1.5% (weight/volume), where the weight is expressed as dry collagen residue and the aqueous solution preferably consists of a weak carboxylic acid or water.

The composition of collagen gels preferably consists of type I, type II, type III collagen or other types of collagen, or is a mixed composition. Type I and/or type II collagen or mixtures of type I and II collagen in different ratios are preferably used, preferably in a ratio 10% to 70% of type II to type I collagen gel. Particularly preferred is the solution comprising a mixture of collagen gels of different types, in which the ratios between the two gel solutions are such that the amount of collagen II is from 10 to 70%(v/v), or better 20-60% (v/v), 30-50%(v/v) and the remainder is type I collagen gel.

Preferably the collagen gel solutions used for preparation of the collagen gel mixture have a collagen concentration from 0.5 to 2 %, preferably between 0.8-1.2% (w/v). Thus, in the final preferred mixture of type I and II collagen, the percentage of type II collagen is from 0.1 to 0.7%, even better 0.2-0.6%, even more preferably 0.3-0.5% (w/v), while the percentage of type I collagen is from 0.5 to 0.7%, 0.4-0.8% or 0.3-0.9% (w/v).

The essential steps, according to the prior art, of the process for collagen gel preparation, including optimization of the alkaline treatment based on the type of collagen used, and of the alkaline treatment are described next:
i) mechanical mincing of a collagen-rich tissue and its homogenization to a collagen suspension,
ii) treatment of the collagen suspension with proteolytic enzymes in acidic pH aqueous solution, optionally followed by filtering,
iii) alkaline treatment of the suspension until a pH preferably higher than 8, and its neutralization with acids to reach a pH preferably comprised between 5 and 6,
iv) collagen precipitation by salting, preferably followed by centrifugation and formation of the collagen gel upon resuspension of the precipitate at a collagen concentration comprised from 0.1 to 5%, preferably from 0.4 to 3%, even more preferably 0.8-1.5% (weight/volume), where the weight is expressed as dry collagen residue in a diluted aqueous solution of a weak carboxylic acid, at a concentration from 0.1 to 1%, preferably from 0.2 to 0.5%, or in water.

Therefore, the starting tissue is preferably a tissue rich in collagen. The tendon is preferably used as the source of type I collagen, even more preferably from horse. Tracheal or joint cartilage is preferably used as source of type II collagen, preferably equine, bovine or swine. Isolated fibroblasts in culture can also be used as source of type I collagen, while cultured chondrocytes can be used as source of type II collagen.

Type III collagen can be isolated from swine skin or from equine, bovine or swine blood vessels. Alternative collagen sources to mammalian connective tissues, for instance fish cartilaginous or bone-cartilage tissue, are well known and usable. Several types of collagens are in fact prepared starting from specific organs or tissues of vertebrates or invertebrate animals, from whole invertebrate organisms, from colonies or agglomerates of animal cells.

According to the present invention, the NaOH treatment, which removes the glycosidic component and possible transmissible contaminating agents and which is mandatory according to the most recent Communitarian Directives (e.g. 75/318/EEC Dec. 1991, EMEA 410/01 rev. 2- October 2003), is performed according to appropriate weight ratios relative to the starting tissue. For tissues rich in type I collagen, the ratio between grams of starting tissue and grams of NaOH is from 0.05 to 0.5, preferably from 0.1 to 0.3, even more preferably from 0.12 to 0.18 or is 0.15; for tissues that are rich in type II collagen, said ratio should not exceed 3.5, and is preferably from 1.8 to 2.8, or even more preferably from 2.1 to 2.5.

Said ratios allow the optimal removal of the immunogenic fraction composed of the glycidic components of the molecule, in particular hexoses, pentoses and hexosamines, while keeping an optimal resistance of the collagen fibers, measured as resistance to enzymatic digestion with collagenases.

After addition of sodium hydroxide in the preferred ratios, NaOH concentration is adjusted to 1 N by addition of an aqueous solvent, preferably water, and the alkaline solution is incubated for at least 1 hour.

The above indicated preferred ratios are especially useful for treatment of type II collagen fibers, which show the highest extent of glycosylation among the collagens that are most commonly used for industrial purposes (I, II, III). It should also be underlined that treatment according to said optimal ratios, defined by the inventors, makes possible to treat with a 1 N sodium hydroxide solution even type II collagen fibers, which show reduced and apparently very weak fibers.

After alkaline incubation, the pH of the collagen suspension is neutralized with a solution of a strong acid: to a pH from 5 to 6, preferably from 5.3 to 5.7 more preferably 5.4-5.6 for type I collagen. Alkalinization and subsequent neutralization, (steps (iii and iv) of the procedure), are very gradual in order to avoid massive precipitation of collagen fibers.

Alternately, when a strong alkalinization of the starting material is either not required, or in other circumstances that are well known to the person skilled in the art, the pH can be adjusted to values comprised between 5 and 6 right after the enzymatic digestion by using a strong base. At this pH (comprised between 5 and 6) type I collagen precipitates upon salting as a whitish mass that can be collected and separated.

The type I collagen precipitate is then washed and resuspended at the most suitable concentration for use (expressed as weight of the dry collagen residue /volume), preferably after removing the precipitated salts by washing with purified sterile water, in a diluted solution of a weak carboxylic acid. Said solution is preferably an acetic acid solution at a concentration from 0.1 to 1 % (w/v), preferably 0.3%. Other weak acids, as for instance citric acid or tartaric acid, but not ascorbic acid, can be used as an alternative to acetic acid, and their concentration is comprised between 0.5-2.5 %, otherwise water can also be used.

Type II collagen fibers are preferably precipitated after alkaline treatment, by adjusting the solution to pH 3.5, with a strong acid, and then adding NaCl at a concentration from 0.65 to 0.75 M, preferably 0.7M.

The precipitated type II collagen fibers can be further concentrated by filtration and/or centrifugation at 4500 rpm for 20 minutes, are subsequently dialyzed to remove salts and then dissolved in a diluted solution of weak carboxylic acid. This acid is even more preferably acetic acid at a concentration from 0.1 to 1 % (w/v), preferably 0.3%. Alternatively, water can also be used.

The precipitation of collagen fibers can be optionally repeated under the same conditions if a cleaner product is desired.

The collagen gel, obtained as described above, can be then homogenized to facilitate resuspension of the precipitate.

Prior to pouring, the gel is preferably warmed up at 24-30°C, and then degassed under vacuum. Other substances can be added to the collagen gel prior to pouring, as for instance biological components of specific body parts or tissues. An example of biological components is represented by polysaccharides of animal or vegetal origin, or matrix proteins such as fibronectin and laminin, or else hormones and growth factors, etc.

### Characteristics of multimicrolamellar collagen membranes.

### Physico-chemical characteristics

The composition of layered collagen gels and the number of layers determine the properties of the membranes: in fact, membranes with different multimicrolamellar structure, biphasic, triphasic and so on, and with different characteristics of resistance and hygroscopy, are obtained by stratification of different types of collagen gels, where for instance the first layer is made of type I collagen and the second of type II collagen or vice versa. In particular, type I collagen multimicrolamellar membranes are much more hygroscopic than type I collagen membranes with the same thickness and prepared according to the prior art by a single pouring and drying (see table 5). The former can absorb a noticeable amount of water, thus swelling until their volume increases 4-5 fold compared to the original volume.

At the same time, such membranes are also more resistant to traction as they have a Young modulus (an index of resistance but also of rigidity of the membrane) that is 7-fold higher compared to membranes known in the art with the same thickness, and a 8-fold higher yielding point.

Type II collagen multimicrolamellar membranes are more hygroscopic than membranes consisting exclusively of type I collagen: they can swell up to a volume that is 7-fold higher that their original volume.
1) the maximum deformability upon traction, that is the maximum degree of stretching reached, is 13 fold less for a membrane composed exclusively by type II collagen compared to a membrane composed exclusively by type I collagen. Increasing amounts of type I collagen result in a deformability that is slightly higher and non-proportional to the amount of type I collagen present in the membranes.
   For a membrane composed exclusively by type II collagen, the Young's module, measured according to methods known in the art, is about 3000-fold lower compared to a membrane composed exclusively by type I collagen. For membranes composed by a mixture of the two types of collagen, and only those containing at least 50% type I collagen, the Young's module reaches a maximum value that is 3-fold higher than the value of type II collagen membranes;
2) the yielding point, that is the maximum load which can be applied, appears to increase with increasing proportions of type I collagen, but it is anyway always lower than 70% in all the membranes that are different from those composed exclusively by type I collagen;
3) the differences among the various types of membrane are less pronounced for the breaking stress, a parameter measuring the load that causes rupture;
4) breaking strain follows a profile that is proportional to the content in type I collagen but is dramatically higher for a membrane composed exclusively by type I collagen than for the others (about 20 - 100 fold, depending on the ratio between the two types of collagen).

Multiphasic membranes, composed by multiple layers each consisting of a single type of collagen, keep the respective characteristics of type I or type II collagen multimicrolamellar membrane. The biphasic membrane, also termed C1+C2, becomes slightly sticky on the surface of type II collagen once it is dipped in water, and is more hygroscopic than a membrane composed exclusively by type II collagen, although it does not reach the level of hygroscopicity of a membrane composed by a 50% mixture of type I and II collagens (C2A-50%).

### Structural characteristics

The membranes, whose preparation is described herein, have different characteristics depending on the type of collagen used and the number and type of stratification performed. However, to our best knowledge, the multimicrolamellar arrangement is novel and original compared to all other membranes that have been obtained according to procedures known in the art, and this arrangement is common to all membranes obtained from collagen gels according to the procedure herein described.

Said structure can be well evidentiated by analysis with the scanning electron microscope (SEM), that reveals a structure of the membranes that is characterized by parallel overlapping microlamellae. Lamellae are formed regardless of the number of sequential additions of collagen gel to the tray: two sequential additions of collagen gel, under the conditions described, are sufficient to obtain a membrane composed of microlamellae. A higher number of additions results in thicker membranes and in a higher content of collagen/cm², thus these membranes are also composed of a higher number of microlamellae.

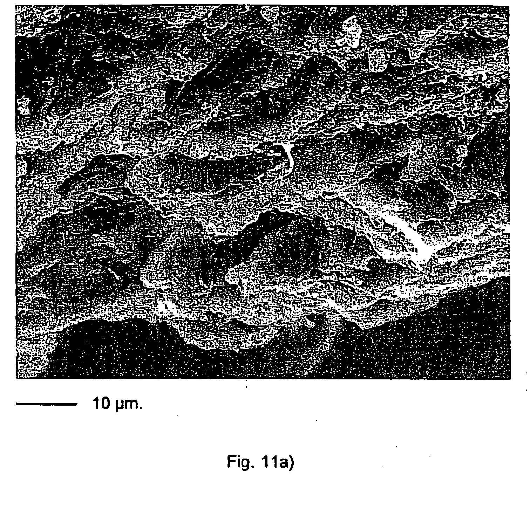

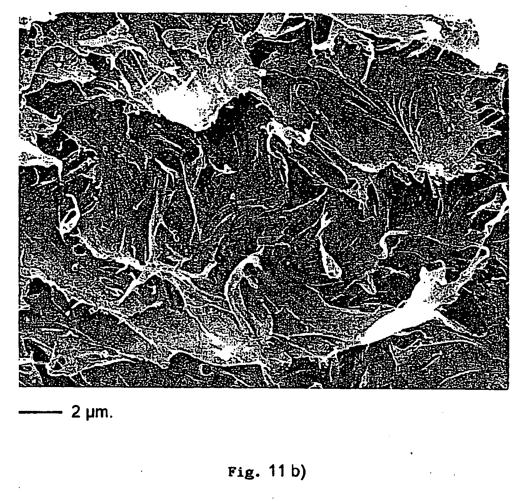

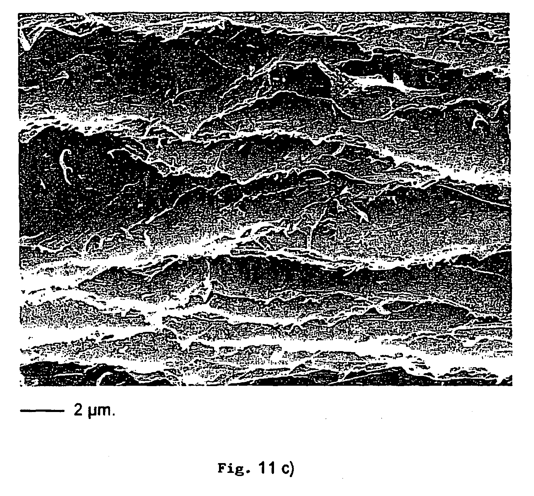

Under the scanning electron microscope, the multimicrolamellar organization of transversely cut type I collagen membranes appears as parallel, overlapping leaflets, which are wide and rather regular but are placed at a close distance that is not constant (see figure 11, a).

The multimicrolamellar organization of transversely cut type II collagen membranes appears to consist of small, roundish, irregular, frayed lamellae that are arranged as fish scales (see figure 11, b)).

Surprisingly, the mixture of type I and II collagen gels results in membranes composed of lamellae that are more homogeneous and regular than those described for type I or II only (see figure 11, c)).

As can be seen also in the SEM images, microlamellae adopt a more ordered structure when the ratio between the two types of collagen is comprised between 20-60% of type II collagen and 80-40% of type I collagen, and membranes with this type of structure are preferred.

Membranes obtained with type I and II collagen mixtures are less resistant to traction than membranes composed exclusively of type I collagen and, surprisingly, are just a little more resistant than membranes composed exclusively of type II collagen, thus they do not show a reliable correlation between proportion of type I collagen and resistance. However they are highly hygroscopic due to the presence of type II collagen: in fact, the incorporation of type II collagen in the organization of type I collagen molecules evidently leads to a different structure of the membrane, making it more easily penetrable by fluids and cells.

### Biological characteristics

In vivo experiments show that multimicrolamellar membranes, preferably the membranes with mixed composition of type I and II collagen, even more preferably those containing a 20-60% proportion of type II collagen, particularly between 40 and 50%, are especially suitable for regeneration of the cartilage tissue.

In fact, *in vivo* repair experiments conducted on animal models reveal that the lesion is populated by cells with characteristics of the cartilaginous lineage already at 3 months after the induction. These cells are apparently viable, they are organized in clusters and are separated by a not really abundant matrix that is intensely basophilic and poorly alcianophilic, indicating the presence of a newly formed matrix whose glycosaminoglycan content is still low.

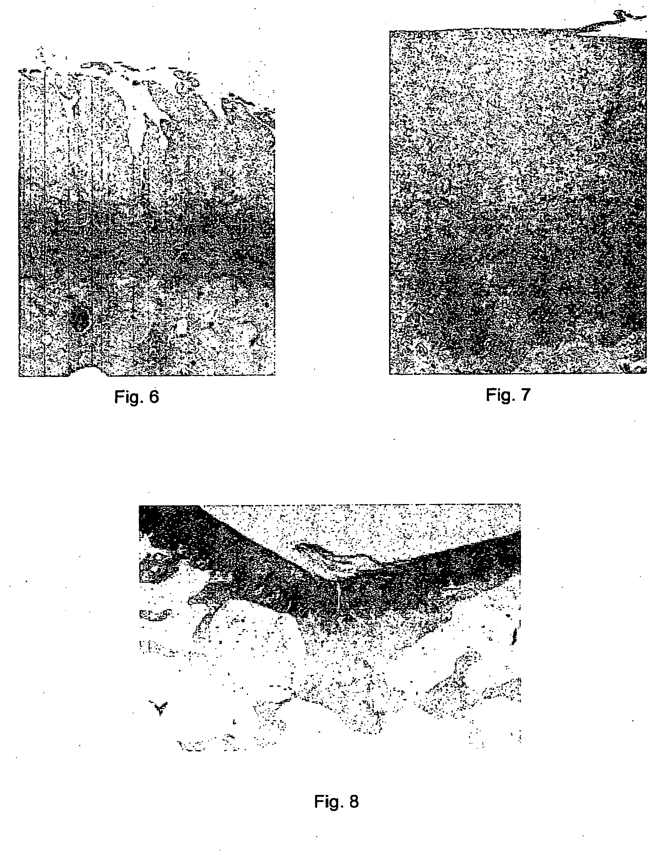

At 9 months, the lesion is filled with repair tissue that is well anchored to the underlying bone and is composed by cartilaginous cells and a newly formed cartilaginous hyaline matrix, as clearly shown in figures 7 and 8. It is possible to observe a tangential arrangement of the most superficial cells and, in the deepest portion of the repair tissue, a normal osteocartilaginous juncture with formation of a clear level or *tide-mark* (Fig. 7 e 8.). Moreover, it is observed that the repair tissue is directly connected to the neighbouring joint cartilage and that the continuity of the articular surface is restored (Fig. 7 and 8).

Histological evidence of cell-mediated immune reactions was not observed in any of the experiments performed. On the contrary, cells attracted to the lesion site and stimulated to grow and/or to differentiate by the membrane three-dimensional support or *scaffold,* give rise to *de novo* formation of cartilage. In this respect, *in vitro* experiments provide evidence that chondrocytes proliferate and maintain their phenotype for a long time when they are cultured on multimicrolamellar membranes composed of type I and II collagen and that pluripotent mesenchymal stromal cells, preferably isolated, proliferate and differentiate as chondrocytes when they are cultured on the same multimicrolamellar collagen membranes with mixed composition (type I and II collagen). Cellular proliferation can be measured, for instance, by the tetrazolium salt test, while chondrocytic differentiation is measured, for instance, by histochemical or immunohistochemical staining with Safranin-O, which primarily reacts with type I collagen and chondroitin sulphate. Multimicrolamellar membranes composed of type I collagen and of type II/I collagen, in a 40/60 ratio, are particularly preferred for both differentiation and proliferation.

In particular, a higher degree of proliferation of adult chondrocytes is observed on the mixed membrane composed of type II/I collagen.

MSC proliferate on the type I collagen multimicrolamellar membrane and differentiate into cells that stain only weakly with Safranin-O and produce type I collagen and chondroitin sulphate. On the contrary, when using the mixed type II/I collagen multimicrolamellar membrane (containing a collagen gel mixture composed preferably of 40% type II collagen and 60% type I collagen), MSC differentiate into chondroblasts showing a clear immune reaction to the S-100 protein, chondroitin sulphate and type II collagen, which are typical reactions of differentiated cartilaginous cells.

The advantages of the membranes according to the invention can be summarized as follows:
1) they prevent the dispersion of osteochondral progenitor cells (mesenchymal, multipotent) from the bottom of the lesion to the articular environment; they optimize membrane cellularization and subsequent cell differentiation *in situ,* thus skipping at least one *in vitro* step compared to the procedure of articular reconstruction with membranes known in the art, that is avoiding the step of cell adhesion and growth on the membrane *in vitro.* This standardizes cellularization with autologous cells;
2) by using membrane, that are permeable to solutes but not to cells, the exchange of solutes required for feeding and proliferation (growth factors, cytokines, etc) of repair cells within the fibrin clot is favoured;
3) in presence of the membranes according to the invention, the repair tissue is optimally integrated with the host tissue, contrasting the anti-adhesive effect observed by some authors that has been attributed to the synovial fluid rapidly "lacquering" the damaged surface.

Various experiments, involving the *in vivo* implant of membranes of the present invention, show that the repair of osteochondral lesions occurs through the following phases:
- adsorption and/or attraction of cells filling the lesion site, which are mostly osteochondral progenitor cells (mesenchymal, that is multipotent of stem type) coming from neighbouring tissues or from perforations of the subchondral bone, according to particular lesion models reproduced in animal models;
- they represent a three-dimensional support that is biologically suited for invasion, proliferation and chondroblastic differentiation of progenitor cells, unlike membranes prepared from collagen gel according to the known art, in which is predominantly observed the formation of a tissue with fibrotic characteristics.

The above described characteristics make these membranes especially suitable for direct use in guided tissue regeneration, without a step of cell expansion in *vitro.* They function as support to the growth of various cell types involved in regeneration, in particular of various types of stem cells such as mesenchymal, stromal and various types of differentiated adult cells. In the case of stem cell, the above described characteristics make these membranes especially suitable also for proper differentiation and regeneration of bone, connective and cartilaginous tissues, depending on the collagen/collagens that compose the membrane.

Therefore the multimicrolamellar membranes according to the invention, preferably those with mixed composition including type I and II collagen, are suitable for repair of cartilaginous lesions, in animals and humans. Such repair is optimized by a subchondral perforation of the bone at the site of lesion performed before placing the membranes of the invention. As mentioned above, such perforation facilitates the attraction of mesenchymal cells from the bone and colonization of the membrane, followed by their *in situ* differentiation in chondrocyte-like cells.

Multimicrolamellar membranes according to the present invention, prepared with the most suitable type of collagens, are used for repair of cartilaginous and bone tissue and for reconstruction of tendons, ligaments and muscle. They are especially useful for the repair of osteochondral lesions or as support membranes.

Therefore the membranes of the invention are especially suitable for reconstruction of osteo-cartilaginous tissue, tendons, bones, joints and as support for non-embryonic stems cells. As described in the experimental part, they function both as physical support and as support capable of inducing differentiation of non-embryonic stem cells in cells of the osteochondral lineage. Therefore, they are also suitable as support for *in vitro* colonization by non-embryonic stem cells.

According to a further aspect, the invention relates to a therapeutic method for repair of cartilaginous lesions, carried out by direct insertion of the membranes in the lesion site, preferably without membrane cellularization (colonization) step and also without cell expansion *in vitro.*

Said therapeutic method is optimized by performing subchondral perforations of the bone before placing the membrane of the invention in the lesion site.

### EXPERIMENTAL PART

### Materials, methods and equipment

### 1) Preparation of collagen and membranes

- Pepsin 700 FIP U/g
- Sterilmixer lab PBI homogenizer
- ALC S2238 refrigerated centrifuge
- Spectrapore 1000 MW dialysis tubings
- Polystyrene trays of various sizes (PBI code 1296, PBI code 4295, PBI code128/8547) exposed to an electrostatic field as described below.
- Rectangular polystyrene spatulas of several widths (9.7 cm, 11.6 cm, 3.2 cm, 4.4 cm, obtained from the above described trays that are cut out in the most suitable sizes).
- "High performance" thermostat Mod. 2800 (FII. Galli G.&P. Milano)
- Electronic outside micrometer IP54 IDF.

*Method of induction of the electrical field:* the trays, completely discharged of electrostatic charge, are placed for 5 minutes between two flat and parallel copper foils connected to a high voltage generator. A static electrical field of about 170kv/m, with vertical orientation, develops between the laminae.

*Method for loading trays for collagen gel stratification:* after charging in the electrical field induced as described above and then deposited on an insulating surface the trays are loaded with the first collagen gel layer, transferred again in the vertical electrical field of 170 kv/m for 5 minutes and then placed in an exsiccator. At the time when a second load of collagen gel is added to the tray, the trays are removed from the exsiccator, transferred on a dispersion surface, loaded with gel, placed in the vertical electrical field of 170kv/m for 5 minutes, then placed again in the exsiccator. Said procedure is also applied to any subsequent collagen gel loads.

### 2) Cell cultures

### - Human dermal fibroblasts

Human dermal fibroblasts have been used, that were isolated from skin biopsies from healthy adult subjects, cultured in Dulbecco's Modification of Eagle's Medium (DMEM) with high glucose concentration, containing L-glutamine 2mM, penicillin 100 UI/ml, streptomycin 100 µg/ml, Na pyruvate 1mM, non essential amino acids (NEAA) 1X and fetal bovine serum (FBS) at 10% final concentration. Fibroblasts were seeded in a two-well "chamber slide" (area = 8.26 cm²), 30000-50000 cells/well, final volume 2 ml. The culture medium was changed every 24 hours.

### - Human chondrocytes

Human chondrocytes were used from biopsies of healthy adult subjects who suffered traumatic lesions. The cell suspension, obtained by trypsinization of a cartilaginous fragment, was washed several times with complete DMEM and chondrocytes were counted with the vital staining method that makes use of eosin, seeded in culture flasks (Costar®, Cambridge, MA, USA) in complete DMEM medium, at a density of 20000/cm², and then placed in a 37°C incubator in humidified atmosphere with 7% CO₂. After adhesion to the support, cells were washed twice with calcium- and magnesium-free phosphate buffer (PBS 1x) and then detached with trypsin/EDTA 1X at 37°C for 10 minutes. The reaction was then blocked with complete DMEM, cells were collected, transferred to large sterile tubes and centrifuged for 5' at 1800 rpm. Two-three ml of complete DMEM were added to the pellet, cells were counted after eosin staining and re-seeded in larger flasks. The medium was changed twice a week.

### 3) Analytical techniques used.

- Determination of the dry residue of collagen gel was performed by a Sartorius MA 50 thermogravimetric balance.
- Electrophoresis by the SDS-PAGE PhastSystem Amersham was performed in Phastgel gradient 4-15% slides.
- Determinations of protein-bound sugars (oses) were performed according to Lusting et. Al, Biochem Z, 242, 320, 1931.
- Determination of NH2 groups was performed according to Bubnis et al., Anal Biochem 207, 129-133,1992.
- Hexosamine determination was performed according to Pieper JS et al., Biomaterials 20, 847-858,1999.
- SEM analysis: the analyses were carried out with a Philips XL-30 Scanning Electron Microscope. Membranes were cut obliquely in 0.5 cm x 0.5 cm pieces (the obliquous cut allows visualization of membrane layers) and fixed to metal *stubs* using carbon disks. Membranes were then metallized with gold, using a metallizer from the Emitech company. Then they were submitted for analysis.
- Analysis of mechanical resistance to traction: it was carried out on 6x25 mm membrane strips using a INSTRON 4465 instrument.
- The test of resistance to collagenases was performed according to Pieper JS, Cross-linked type II collagen matrices: preparation, characterization, and potential for cartilage engineering. 23, 3183-3192, 2002.

### Example 1. Preparation of type II collagen from cartilage and purification with sodium hydroxide.

50 g of equine joint cartilage powder were repeatedly washed in water, drained and placed in 3 liters of water that were brought to pH 2.5 with hydrochloric acid solution. Five grams of pepsin were added to the suspension (10% of the starting cartilage) under mechanical stirring.

After about 20 hours at about 27 °C, the suspension was again stirred repeatedly, filtered, and the fluid was collected, diluted to 3.75 liters and adjusted to pH 3.5 with few drops of a 2.5 N sodium hydroxide solution. 154.85 g of sodium chloride was added to the fluid to a concentration of 0.7M : after about 2 hours, a precipitate was formed consisting of very short (few mm) and thin fibers. The precipitate was collected on a cotton cloth by filtration under vacuum and subsequent centrifugation at 4500 rpm for 20 minutes, until a very concentrated suspension was obtained. An aliquot of the suspension (28 g) was dialyzed against water in a Spectrapore 1000 MW tubing for 65 hours at 4°C. At the end of dyalisis, the sample was brought to 100g with 0.3% acetic acid and was designated C2/030717. Instead, other 300 g of the concentrated fiber suspension were treated with 20 g of sodium hydroxide and brought to 500 ml (1N sodium hydroxide solution). The ratio between starting cartilage (about 46 gm after removing the first aliquot of 28 g of concentrated fiber suspension) and sodium hydroxide was calculated to be 46g/20g=2.3. Fibres completely dissolved were kept in sodium hydroxide for 1 hour at about 23 °C. After diluting with 1.5 liters of water and adjusting the pH to 3.5 with HCI, a new precipitation of fibers was obtained in about two hours. The new fibers, collected by centrifugation, were dyalized in Spectrapore 1000 MW tubings in water. A 90 % proportion of the so obtained sample, already in the form of a gel that was not very viscous, was dissolved in a few milliliters of acetic acid at 0.3% final concentration and was designated C2/030718 (628 g). The other 10% was dissolved in a proportional amount of water: a gel was obtained also in this case, that was designated C2/030718H (about 50 g).

C2/030717 and C2/030718 collagens were electrophoretically (PAA 4-15%) identical to a commercial type II collagen that was taken as reference (SIGMA C-1188). C2/030718 was cleaner than C2/030717 which is shown by the low molecular weight electrophoretic bands which are completely removed by sodium hydroxide treatment.

Table 1 shows the characteristics of the 2 samples:

**Table 1**

| Sample | NH₂M/g*10^5 | "oses" µg/mg | % hexosamine |
|---|---|---|---|
| C2/030717F | 37.8 | 27.0+/-1.2 | 0.63 |
| C2/030718F | 38.3 | 22.8+/-1.1 | 0.03 |

Free NH2 groups remain almost unchanged, while sodium hydroxide treatment removes a fraction of sugars and hexosamines. Table 2 shows several tests of treatment of type II collagen fibers with sodium hydroxide.

The table shows various ratios between the starting cartilage and sodium hydroxide in relation to the characteristics of the resulting collagen (presence of sugars, percentage of hexosamines, resistance of the molecule to collagenases).

Sodium hydroxide treatment of type II collagen gives optimal results when the ratio between the starting tissue and sodium hydroxide is comprised between 2 and 3.5 and the ratio between grams of tissue/ml is comprised between 0.08 and 0.15. It should be noted that membranes from lots 030718 and 050415 are more resistant to the attack by collagenases at a concentration of 10U/ml at 37°C.

**Table 2. Optimization of the treatment of type II collagen fibers with sodium hydroxide**

| Lot of type II collagen | cartilage (g) | NaOH (g) | vol. (ml) | Cartilage (g/ml) | 1N NaOH sin (g/ml) | cartilage /NaOH | break-up in collagenase (hrs) | "ose" sugars. (µg/mg) | hexosamine % |
|---|---|---|---|---|---|---|---|---|---|
| 020305ppt | | 0 | | | | | 6 | | |
| 30717 | | 0 | | | | | | 27 | 0.63 |
| 020305NaOH | 6.29 | 9.45 | 290 | 0.02 | 0.03 | 0.7 | 1 | | |
| O20916 | 22 | 20 | 500 | 0.04 | 0.04 | 1.1 | 5 | 21.3 | |
| O30116 | 11 | 8 | 200z | 0.06 | 0.04 | 1.4 | | 22.9 | |
| O30718 | 46 | 20 | 500 | 0.09 | 0.04 | 2.3 | 30 | 22.8 | 0.03 |
| O40120 | 76.33 | 38 | 954 | 0.08 | 0.04 | 2.0 | | | |
| O50415st | 27.18 | 8 | 200 | 0.14 | 0.04 | 3.4 | 6 | | |
| C2A100-050415 | 27.18 | 8 | 200 | 0.14 | 0.04 | 3.4 | 20 | | |
| O50615 | 45.88 | 8 | 200 | 0.23 | 0.04 | 5.7 | 6 | | |
| O50708/B | 79 | 15.9 | 400 | 0.20 | 0.04 | 5.0 | | | |

| | | | | | | | | | |
|---|---|---|---|---|---|---|---|---|---|
| NaOH=sodium hydroxide | | | | | | | | | |

Table 3 shows the evaluation of mechanical resistance to traction of the membrane obtained from collagen 030718 compared to that obtained from 050615 (C2A050617P100 membrane).

**Table 3**

| Lot membrane Type II collagen | Collagen mg/cm² | Max Def % | Yelding point (Mpa) | Young module (Mpa) | breaking stress (Mpa) | Breaking strain % |
|---|---|---|---|---|---|---|
| C2A030718 | 9 | 6.043 | 0.045 | 0.016 | 5.588 | 0.99 |
| C2A050617P100 | 9 | 2.86 | 0.007 | 0.003 | 2.767 | 0.31 |

Mpa: 1 megapascal (MPa) = 1 000 000 Pa = 1 N/mm². Def: deformability Collagen 050615 treated with sodium hydroxide at a concentration that is too low relatively to the starting cartilage (cartilage/sodium hydroxide=5.7) gives rise to a membrane (C2A050617P100) that is less resistant to traction compared to that obtained with collagen 030718.

### Example 2. Preparation of type I collagen from equine tendon and purification with sodium hydroxide.

An amount of 80 g of ground equine tendon was placed in 1 liter of water, left to swell for two hours, homogenized in a Sterilmixer Lab homogenizer. PBI : 0.81 g of pepsin were added to the suspension that was then brought to 10 liters with water. The pH was adjusted to 2.5 and the suspension was left for 23 hours at 20°C +/-2°C. After this time the suspension was again homogenized with a Sterilmixer and filtered on cotton cloth. The liquid filtrate was adjusted to pH 5.5 with 30% sodium hydroxide, resulting in precipitation of collagen fibers that were washed twice with water at pH 6. An amount of 304.55 g of wet fibers was recovered. From this material, 50 g were washed with water 2 more times and then dissolved in 0.3% acetic acid (they were brought to 180 g in order to obtain a 1% collagen concentration : the so-formed gel (050920 A) was repeatedly homogenized with a Sterilmixer and was degassed under vacuum and under mechanical stirring. Fibers left after removal of the 50 g aliquot (253.9 g) were treated with 1540 ml of 30% sodium hydroxide and brought to 12 liters (in order to obtain 1 N sodium hydroxide concentration) for 1 hour. Under the conditions used, the ratio between grams of starting organ (tendon) and sodium hydroxide is about 0.5. This tendon/sodium hydroxide ratio turned out to be optimal for purification of type I collagen suitable for preparation of multimicrolamellar membranes.

After one hour incubation, the solution was brought to pH 5.5 with HCI, thus resulting in re-precipitation of fibers. These were drained, washed 3 times with water at pH 6 and then recovered with 0.3% acetic acid in an amount suitable to obtain 798 grams of 1% collagen gel (lot 050920 AN).

### Example 3. Isolation of collagen from dermal fibroblasts cultured in vitro.

Human dermal fibroblasts isolated from skin biopsies of adult healthy subjects were cultured in plastic wells according to the conditions described in the section "materials and methods". The cell culture medium was replaced according to a pre-arranged schedule, so that cells were always fed with fresh medium. The medium withdrawn from wells every 24 hours was collected and frozen at -20°C. Aliquots kept at -20°C were thawed and pooled together: in total, 102.66 ml of culture medium were collected, containing products of cellular metabolism. The fluid was subjected to a process for the extraction of type I collagen, following the method described in example 2, with an appropriate resizing of the procedure.

At the end of the process, 19.4 mg of wet fibers were obtained (which turned out to consist of type I collagen, by polyacrylamide gel electrophoresis) that were recovered in 50 microliters of 0.3% acetic acid : a viscous and homogeneous gel was formed in few minutes.

### Example 4. Isolation of collagen from chondrocytes cultured in vitro.

Human chondrocytes, isolated from joint cartilage of adult healthy subjects who have undergone several type of surgical interventions following traumas, were cultured in plastic wells according to the conditions described in the section "materials and methods". The cell culture medium was replaced according to a pre-arranged schedule, so that cells were always fed with fresh medium. The medium withdrawn twice a week was collected and frozen at -20°C. Aliquots kept at -20°C were thawed and pooled together: in total, 77.23 ml of culture medium were collected, containing products of cellular metabolism. The fluid was subjected to the process for extraction of type II collagen, following the method described in example 1, with the appropriate scale down. At the end of the process, 7.89 mg of very short and light wet fibers were obtained (which turned out to consist of type II collagen, by polyacrylamide gel electrophoresis) that were recovered in 25 microliters of 0.3% acetic acid: a clear, fluid and homogeneous gel was formed in few minutes.

### Example 5. Preparation of multimicrolamellar membranes from type I collagen gels

All the trays used for preparation of the membranes of this example were exposed to an electrostatic field according to the procedure described in the section "Materials, methods and equipment"

### a) membrane with 9 mg of collagen/cm² lot 40122/1

Membranes were prepared from a 1% collagen gel, equilibrated at 26°C+/-2°C. An amount of 65.5 g of 1% collagen gel, lot 050920 AN, was poured in a square polystyrene tray having a side of 12 cm, and left to expand until a homogenous layer covered the bottom completely. The gel was spread with a 11.6 cm wide spatula made of the same material as the tray, until a homogenous layer covered the bottom completely. The edge of the spatula was passed several times on the gel. The thickness of this layer was 5.52 mm, as measured by an Electronic outside micrometer IP54 IDF. Time was recorded as T01 upon the first stratification. The tray was placed in thermostat at constant temperature of 26°C+/-2°C, with ventilation set to the minimum, until its thickness was reduced to 2.85 mm after 48 hours, due to evaporation. At this point, an amount of gel identical to the first amount was again poured in the tray, covering completely and homogeneously the semi-solid surface of the previous layer: also in this case the gel was spread with a 11.6 cm wide spatula made of the same material as the tray, until a homogenous layer covered the bottom completely. The edge of the spatula was passed a few times on the gel. The total thickness of the new semi-solid membrane (6.36 mm) was recorded at the new T0 (T02) and subsequently controlled over time, monitoring the thickness every 24 hour, while the system was kept at 26°C+/-2°C without ventilation. At this point the membrane was kept under the same temperature conditions and without ventilation until complete exsiccation and spontaneous detachment from the bottom of the template.

### b) membrane with 14 mg of collagen/cm² lot 40122/2

In a square polystyrene tray having a side of 12 cm, 65.5 gm of 1.56% collagen gel, lot 40121D, were poured. The membrane was prepared according to the procedure described in a).

### c) membrane with 18 mg of collagen/cm² lot 40122/3

Membranes were prepared according to the procedure described in a), starting with 130 g of 1% collagen gel (lot 050920 AN), equilibrated at 26°C+/-2 and poured in a square polystyrene tray having a side of 12 cm.

### d) membrane with 23 mg of collagen/cm² lot 40122/4

Membranes were prepared starting from 100 g of 1.11 % collagen gel, that was equilibrated at 26°C+/-2°C and poured in a square polystyrene tray having a side of 12 cm. The total thickness of the new semi-solid membrane (8.12 mm) was recorded at the new T0 (T02) and subsequently controlled over time, monitoring the thickness every 24 hour, while the system was kept at 26°C+/-2°C with minimal ventilation.

Onto the 4.26 mm thick sheet (72 hours after the last gel addition) a third amount of gel, having a weight identical to the previous two amounts, was homogenously distributed on the semi-solid surface of the underlying layer, always using a polystyrene spatula. Again the thickness of the semi-solid membrane was recorded as T03 (9.06 mm). At this point the membrane was kept under the same temperature conditions and without ventilation until complete desiccation and spontaneous detachment from the bottom of the template.

Examples a, b, c, d indicate that the characteristics of the membrane can be modulated by changing gel concentration and number of layers. The table below shows the absorption capacity of each membrane of example 3 within 24 hours.

Table 4 shows the increase in thickness (mm) over time of the above described membranes dipped in water.

**Table 4**

| **Lot** | **mg/cm²** | **T0** | **15 min** | **30 min** | **60 min** | **24 h** |
|---|---|---|---|---|---|---|
| **40122/1** | 9 | 0.113 | 0.519 | 0.516 | 0.533 | 0.632 |
| **40122/2** | 14 | 0.148 | 0.568 | 0.658 | 0.777 | 0.966 |
| **40122/3** | 18 | 0.187 | 0.641 | 0.762 | 0.851 | 1.163 |
| **40122/4** | 23 | 0.220 | 0.782 | 0.856 | 0.949 | 1.576 |

### Example n. 6. Preparation of membrane with 19 mg of collagen/cm² - lot 40126

Membranes were prepared starting from a 1.06 % collagen gel, lot 78802, prepared as in example n.2. The gel was subjected to deaeration (to exclude air bubbles) by passage in a suitable planetary mixer under vacuum and stirring for 24 hours, then it was kept under vacuum without stirring for additional 12 hours, followed by equilibration at 28°C+/-2°C. An amount of 130 g of collagen gel was poured in square polystyrene trays having a side of 12 cm, exposed to an electrical field as described in materials and methods, paying attention not to form air bubbles. Using the above described spatula, the gel was distributed in each tray until a homogenous layer, with an average thickness of 10.52 mm, covered the bottom completely. The trays were kept at a constant temperature of 26°C+/-2°C, with ventilation set to the minimum, until their average thickness was reduced to 5.55 mm after 46 hours, due to evaporation. At this point, an amount of gel identical to the first amount was again poured in each tray, following the procedure already described. The new average total thickness of the semi-solid membranes was 14.11 mm. The system was kept at 26 °C without ventilation until complete spontaneous detachment of the exsiccated membranes from the trays. By SEM analysis, the resulting membranes, with a homogeneous thickness of 0.25 mm, appear to be composed of several parallel microlamellae.

Membrane 40126 swells in water up to a 4-fold increase of thickness.

The graph in table 5 shows the increase in thickness of membrane 40126 in water compared to a membrane prepared according to EP1307247, with the same initial thickness (lot 40124).

**Table 5.**

| | | **Thickness mm** | | | |
|---|---|---|---|---|---|
| membrane | type | T0 | 15 min | 30 min | 60 min |
| lot 40124 | EP1307247 | 0.258 | 0.590 | 0.642 | 0.684 |
| lot 40126 | multimicrolam | 0.250 | 0.698 | 0.870 | 1.056 |

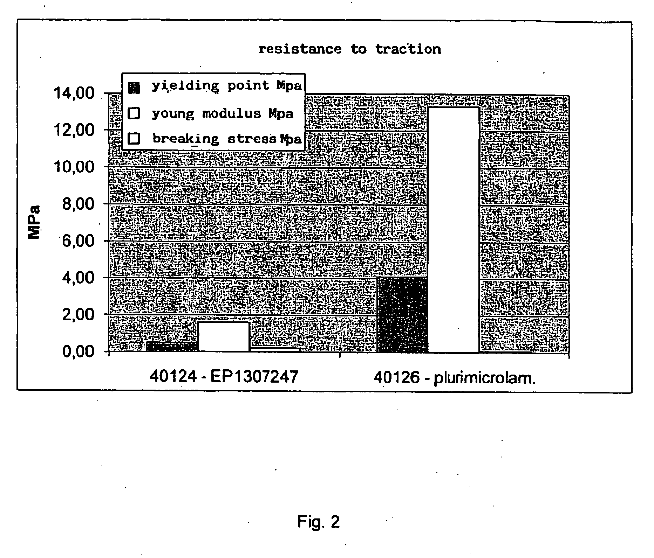

Moreover, the multimicrolamellar membrane 40126 is more resistant to traction than 40124, as shown in the histogram in figure 2.

### Example 7. Type II collagen multimicrolamellar membrane (lot 030718)

Membranes were prepared starting from a 1% gel composed of type II collagen (lot 030718), equilibrated at 18°C+/-2. An amount of 65.5 g of collagen gel was poured in a square polystyrene tray having a side of 12 cm, exposed to an electrical field, as described in materials and methods, and distributed with a spatula made of the same material as the tray, forming a 5.02 mm thick layer. The tray was incubated at a constant temperature of 24°C+/-2°C, without ventilation, until the thickness was reduced to 1.89 mm in 48 hours, due to evaporation (the thickness was controlled every 24 hours). At this point an amount of gel equal to the first amount was again poured in the tray, covering completely and homogeneously the semi-solid surface of the previous layer, whose surface was rendered homogeneous with the polystyrene spatula described previously. The total thickness of the new semi-solid membrane (6.36 mm) was monitored over time, performing controls every 24 hours, keeping the system at 24°C+/-2°C without exsiccation until complete exsiccation and spontaneous detachment from the bottom of the tray.

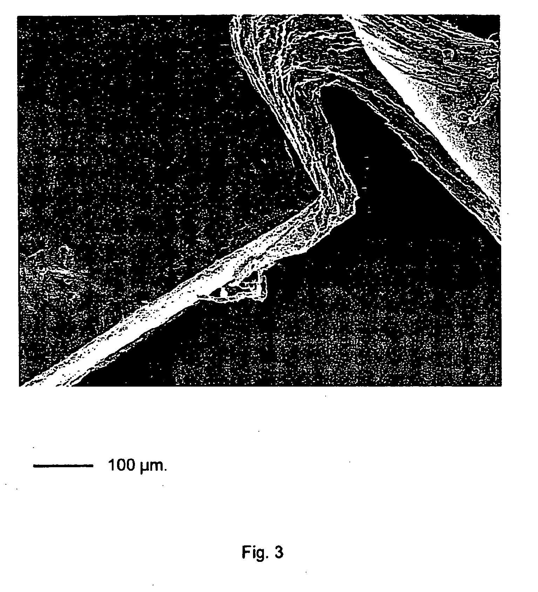

The membrane (9 mg/cm²) was marked C2A-030718 (figure 3 shows the scanning electron microscope analysis).

The weight and the thickness of membrane C2A-030718 and of the reference type I collagen membrane C1-78908, comparable by collagen content per cm², were assessed before and after water immersion for 1 hour.

**Table 6.**

| **membrane** 9 mg/cm² | Collagen type | Weight i (mg) | Weight f (mg) | Δ weight (mg) | Thickness i (mm) | Thickness f (mm) | Δ thickness (mm) |
|---|---|---|---|---|---|---|---|
| C1-78908 | I | 12.7 | 37.3 | 24.6 | 0.100 | 0.297 | 0.2 |
| C2A-030718 | II | 9.8 | 81.5 | 71.7 | 0.127 | 0.767 | 0.6 |

| | | | | | | | |
|---|---|---|---|---|---|---|---|
| i= initial f=final | | | | | | | |

Compared to a similar type I collagen membrane, the membrane C2A-030718 is very hygroscopic, as shown by the data in table 4.

### Example 8. Mixed type I collagen I type II collagen multimicrolamellar membranes

A few mixtures of type I and type II collagen, in various proportions, have been prepared, starting from the type II collagen gel lot C2/030718 (see example n.2) and from the type I collagen gel lot 78791 (both of which are about 1 %). The two gels were equilibrated at 22°C+/-2, and mixed in several proportions:
5 g of C2A/030718 + 95 g of 78791 = 5% type II collagen
10 g of C2A/030718 + 90 g of 78791 = 10% type II collagen
20 g of C2A/030718 +80 g of 78791 =20% type II collagen
40 g of C2A/030718 + 60 g of 78791 =40% type II collagen
60 g of C2A/030718 + 40 g of 78791 =60% type II collagen
80 g of C2A/030718 + 20 g of 78791 =80% type II collagen

From the above mentioned mixtures, a corresponding number of membranes were prepared by superimposition of two sequential gel loads, as described in example 7.

By SEM analysis, microlamellae appear large and distant one from the other: this structure allows an optimal absorption, that is three-fold higher compared to membranes consisting exclusively of type I collagen and one and half-fold higher compared to membranes consisting exclusively of type II collagen (table 7).

**Table 7**

| LOT membranes | Weight i (mg) | Weight f (mg) | Δ weight (mg) | Thickness i (mm) | Thickness f (mm) | Δ thickness (mm) |
|---|---|---|---|---|---|---|
| C2A-100% | 9.8 | 81.5 | 71.7 | 0.127 | 0.767 | 0.6 |
| C2A-40% | 14.0 | 136.4 | 122.4 | 0.140 | 1.000 | 0.9 |
| C2A-5% | 5.3 | 16 | 10.7 | 0.150 | 0.217 | 0.1 |
| C1-100% | 12.7 | 37.3 | 24.6 | 0.100 | 0.297 | 0.2 |

| | | | | | | |
|---|---|---|---|---|---|---|
| i: initial; f: final | | | | | | |

From table 7, it turns out that membranes obtained from mixtures of type I collagen and type II collagen do not have predictable behaviour when they are evaluated with respect to weight and thickness before and after imbibition in water for one hour. In particular, a membrane containing 5% type II collagen (C2A-5%) gives rise to a more compact and less hygroscopic membrane compared to a membrane composed exclusively of type I collagen (C1-100%), while 40% type II collagen (C2A-40%) renders the membrane even more suitable for fluid absorption than a membrane composed exclusively of type II collagen (C2A-100%).

### Example 9. Mixed type I collagen / type II collagen multimicrolamellar membranes.

A few mixtures of type II and type I collagen, in various proportions, have been prepared, starting from the type II collagen gel lot C2/050415 and from the collagen gel lot 78908 (both of which are about 1 %). The two gels were equilibrated at 20°C+/-2°C, and mixed in several proportions:
0 g of C2A/050415 + 100 g of 78908 = 100% type I collagen
25 g of C2A/050415 + 75 g of 78908 = 25% type II collagen
50 g of C2A/050415 + 50 g of 78908 = 50% type II collagen
75 g of C2A/050415 + 25 g of 78908 = 75% type II collagen
100 g of C2A/050415 + 0 g of 78908 = 100% type II collagen

From the so prepared mixtures, membranes were obtained that were prepared as in example 7.

**Table 8**

| LOT membranes | Weight i (mg) | Weight f (mg) | Δ weight (mg) | Thickness i (mm) | Thickness f (mm) | Δ thickness (mm) |
|---|---|---|---|---|---|---|
| C2A-100% | 9.8 | 81.5 | 71.7 | 0.127 | 0.767 | 0.64 |
| C2A-75% | 10.4 | 53.7 | 43.3 | 0.110 | 0.477 | 0.37 |
| C2A-50% | 13.7 | 109.7 | 96.0 | 0.103 | 0.993 | 0.89 |
| C2A-25% | 13.6 | 51.8 | 38.2 | 0.117 | 0.477 | 0.36 |
| C1-100% | 12.7 | 37.3 | 24.6 | 0.100 | 0.297 | 0.20 |

| | | | | | | |
|---|---|---|---|---|---|---|
| i: initial; f: final | | | | | | |

Table 8 shows the weight and the thickness of mixed type I+II collagen membranes in different proportions before and after imbibition in water for one hour. Clearly, the more organized structure, which is obtained with a percentage of type II collagen of about 50%, leads to a better predisposition of the membrane for imbibition (see also the example 6, where the sample containing 40% type II collagen is morphologically more regular and organized and functionally more hygroscopic).

### Example 10. Preparation of the type II collagen micromultilamellar membrane from trachea (lot C2T/100/1)

Membranes were prepared starting from 1.03% type II collagen gel (lot 050627, containing traces of type I collagen, prepared following the method described in example n. 1, by using a cartilage/sodium hydroxide ratio = about 2, calculated from the presumed amount of cartilage in the starting trachea) that was equilibrated at 22°C+/-2°C. Also in this case, membranes were prepared with two subsequent gel additions according to the procedure described in example n.5. The membrane lot has been identified with the C2T/100/1 abbreviation. By scanning electron microscopy, also this membrane turned out to consist of parallel microlamellae.

### Example 11. Preparation of type II collagen multimicrolamellar membrane (lot 030718H).

The membrane was prepared in a round polystyrene plate of 6 cm diameter, starting from type II collagen gel lot 030718H, that was obtained by dissolving collagen fibers in water rather than acetic acid. The preparation method is the same as described in example 7.

The resulting membrane, 0.14 mm thick, is very fragile once hydrated, but is able to swell up to 1.22 mm thickness after 1 hour (almost 9-fold compared to the initial thickness). The swollen membrane is very sticky.

### Example 12. Preparation of type I collagen + type II collagen biphasic membrane.

The membrane was prepared starting from 1.15 % type I collagen gel, lot 78791, that was prepared as in example 2, and from 1.07% type II collagen gel, lot C204120, that was prepared as in example 1. An amount of 130 g of collagen was poured in a square polystyrene tray having a side of 12 cm, exposed to electrostatic field as described in materials and methods, paying attention not to form air bubbles. The gel was spread to form a homogenous layer, using a spatula made of the same material as the tray. The thickness of this layer was 10.46 mm. The tray was incubated at constant temperature of 24°C+/-2°C, with ventilation set to the minimum, until its thickness was reduced to 5.39 mm after 72 hours, due to evaporation. At this point, 130 g of 1.07% type II collagen gel (lot C204120) were poured in the tray, covering completely and homogenously the semi-solid surface of the previous layer. Also in this phase the gel was stratified homogeneously with the polystyrene spatula. The new thickness of the semi-solid membrane (14.23 mm) was monitored over time, performing controls of the thickness every 24 hours, keeping the system at 26°C+/-2°C, without ventilation, until complete exsiccation and spontaneous detachment from the bottom of the template. The membrane, termed C1+C2, becomes slightly sticky on the surface of type II collagen once it is dipped in water, and is more hygroscopic than a membrane composed exclusively by type II collagen, although it does not reach the level of hygroscopicity of a mixed membrane composed by a 50% mixture of type I and II collagens (C2A-50%).

**Table 9**

| LOT | Weight i (mg) | Weight f (mg) | Δ weight (mg) | Thickness i (mm) | Thickness f (mm) | Δ thickness (mm) |
|---|---|---|---|---|---|---|
| C2A-100% | 9.8 | 81.5 | 71.7 | 0.127 | 0.767 | 0.64 |
| C2A-50% | 13.7 | 109.7 | 96.0 | 0.103 | 0.993 | 0.89 |
| C1+C2 | 12.4 | - | - | 0.203 | 0.942 | 0.74 |
| C1-100% | 12.7 | 37.3 | 24.6 | 0.100 | 0.297 | 0.20 |

| | | | | | | |
|---|---|---|---|---|---|---|
| i: initial; f: final | | | | | | |

Table 9 shows the data relative to the swelling in water of the bilayer type I + type II collagen membrane (C1+C2) compared with membranes consisting exclusively of type I collagen (C1-100%), type II collagen (C2A-100%), and the data relative to the swelling in water of the membrane obtained from a 50% mixture of type I and II collagen gels (C2A-50%).

### Example 13. Measurement of the mechanical resistance of type I collagen / type II collagen mixed membranes

With respect to various parameters that have been considered, membranes composed exclusively by type II collagen are much less resistant to traction than membranes composed exclusively by type I collagen.

Membranes obtained by mixing the two collagens in various proportions have unpredictable mechanical characteristics: the presence of type I collagen results in little and not always proportional (see Young's module) increase of membrane resistance.

**Table 10**

| Type of membranes Type of collagen | II | I | Coll. mg/cm² | Max def % | Yielding point (Mpa) | Young modulus (Mpa) | breaking stress (Mpa) | Breaking strain % |
|---|---|---|---|---|---|---|---|---|
| 0617P100 | 100 | 0 | 9 | 2.86 | 0.007 | 0.003 | 2.767 | 0.31 |
| C2A050617P/mix75% | 75 | 25 | 9 | 3.728 | 0.012 | 0.003 | 3.661 | 0.458 |
| C2A050617P/mix50% | 50 | 50 | 9 | 4.060 | 0.023 | 0.009 | 3.881 | 0.860 |
| C2A050617P/mix25% | 25 | 75 | 9 | 2.937 | 0.029 | 0.008 | 2.878 | 1.471 |
| membrane 40091 | 0 | 100 | 9 | 39.00 | 2.150 | 10.000 | 2.000 | 35.000 |

| | | | | | | | | |
|---|---|---|---|---|---|---|---|---|
| Max % def deformability measured as described in materials and methods | | | | | | | | |

From the data shown in table 10, it can be observed that:
1) the maximum deformability upon traction, that is the maximum degree of stretching, is 13 fold less for a membrane composed exclusively by type II collagen compared to a membrane composed exclusively by type I collagen. Increasing amounts of type I collagen results in a deformability that is slightly higher and non-proportional to the amount of type I collagen present in the membranes.
   For a membrane composed exclusively by type II collagen, the Young modulus (an index of resistance but also of rigidity) is about 3000-fold lower compared to a membrane composed exclusively by type I collagen. For membranes composed by a mixture of the two types of collagen, and only those containing 50% and 75% type I collagen, the Young modulus reaches a maximum value that is 3-fold higher than the value of the type II collagen membrane.
2) yielding point, which is the maximum load that can be applied, appears to increase with increasing proportions of type I collagen, but anyway it is always lower than 70% in all the membranes that are different from those composed exclusively by type I collagen.
3) the differences among the various types of membrane are less marked for the breaking stress, a parameter measuring the load that causes rupture.
4) breaking strain follows a profile that is proportional to the content in type I collagen but is dramatically higher for a membrane composed exclusively by type I collagen than for the others (about 20 - 100 fold, depending on the ratio between the two types of collagen).

### Example 14. Structure definition by scanning electron microscopy of membranes obtained from mixtures of type I collagen and type II collagen.

Membranes obtained from mixtures of type I collagen and type II collagen were evaluated for their microlamellar structure with a scanning microscope (SEM) at high magnification.

Figure 11a shows a SEM analysis at 2500 x magnification of the 100% type I collagen multimicrolamellar membrane (40126). Fig. 11b shows a SEM analysis at 5520 x magnification of the 100% type II collagen multimicrolamellar membrane (030718).

Images show that type I collagen microlamellae are thick and further stratified, while those of type II collagen are thin, frayed and disorderly arranged.

Figure 11c shows a SEM analysis at 5030x magnification of a mixed multimicrolamellar membrane composed by 40% type II collagen (C2 40%). Mixing the two collagens in the proportion 40% type II - 60% type I leads to a better organization of the membranes in ordered and defined microlamellae.

### Example 15. Effect of multimicrolamellar membranes on in vivo cartilage regeneration in rat

Type I collagen multimicrolamellar membranes, type I/II collagen mixed multimicrolamellar membranes and biphasic type I collagen + type II collagen membranes were tested in rat for a preliminary experiment on direct cartilaginous regeneration without a step of *in vitro* expansion of autologous chondrocytes, and they were compared with type I collagen monolayer membranes. The method is described in the section "Materials, methods and equipment".
1 st group: in the right knee, the type I collagen membrane, lot 40124, prepared according to EP1307247.
2nd group: in the right knee, the type I collagen multimicrolamellar membrane, lot 40126.
3rd group: in the right knee, the mixed type II/I collagen multimicrolamellar membrane (40% and 60%, respectively), lot C2A40.
4th group: in the right knee, the biphasic type I+II collagen membrane, lot C1+C2, with the type II collagen surface facing the lesion and the type I collagen surface facing the articular cavity. In this group, it was not necessary to fix the membranes with type I collagen gel, because the type II collagen surface, that was slightly gellified after immersion in physiological solution, was sticking to the lesion borders.

Lesions of the left knee (control) were evident in all animals. The lesion bottom did not appear very different at time 0, even if a little filling layer was clear in a few cases. On average, the reconstruction was 0.02 mm.

In the group treated with 40124, defects were evident and the cartilage appeared only partially reconstituted and irregular at the bottom of the lesion. On average, the thickness of the cartilage in the lesion was 0.05 mm.

In the group treated with 40126, defects were also evident but the cartilage appeared reconstituted to a certain extent, though irregularly in a significant portion of the lesion. On average, the thickness of the cartilage in the lesion was 0.12 mm.

In the group treated with C2A40, the defects were almost completely filled with tissue very similar to the surrounding cartilage. On average, the thickness of the cartilage in the lesion was 0.36 mm, and the surface was quite regular.

In the group treated with C1+C2, reconstitution was very similar to that observed with C2A40. On average, the thickness of the cartilage in the lesion was 0.30 mm, and the surface was quite regular.

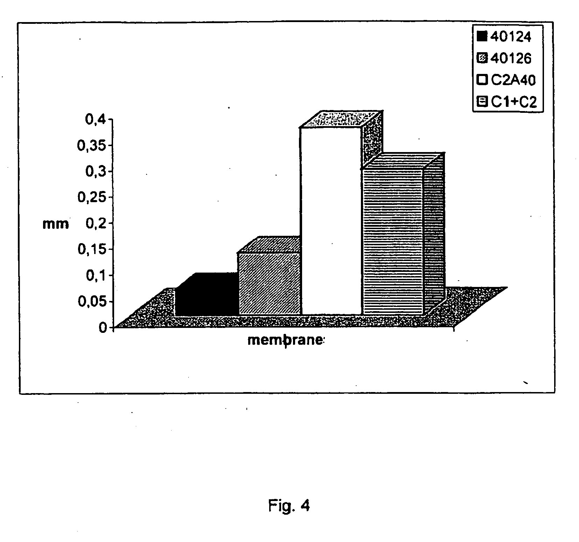

Figure 4 shows the histogram of the thickness of joint cartilages in the damaged area after 6 months from treatment with type I collagen membranes prepared according to EP1307247 (40124), with type I multimicrolamellar collagen (40126), with mixed type I/II multilamellar collagen (C2A40) and with two distinct layers of type I collagen and type II collagen (C1+C2), showing that cartilage repair is faster in presence of membranes with multimicrolamellar or mixed structure.

### Example 16. Repair of osteochondral lesions by Pridie perforations and implant of three-dimensional matrix based on type I/II collagen: in vivo experimental study.

The *in vivo* cartilage regeneration test was carried out according to the method of Pridie (Pridie K. H. A Method of resurfacing Osteoarthritic Knee Joints. J. Bone Joint Surg., 1959, 41 -B, 618-619) both in rat and sheep.

Under conditions of surgical sterility, a longitudinal side cut of soft tissues and of the articular capsule was made in all animals, which had been subjected to general anaesthesia and, after medial dislocation of the rotula, the medial femoral condyles of the right and left femur were exposed in order to inflict the cartilaginous lesion. Deep perforations were made at the bottom of every lesion, to allow the penetration of mesenchymal cells coming from the bone underlying the site of lesion. The lesion of the left knee of each animal was not treated, while the lesion on the right was filled with the various membranes according to the group.

In the rat, the membranes, after hydration in physiological solution, were fixed to the lesion borders with a thin layer of 2% type I collagen gel. In the sheep, instead, membranes were sutured. At last, the rotula was placed back in position, and the articular capsule and the skin were sutured.

After 3, 6 and 9 months, animals were euthanized, femurs were explanted and cartilaginous lesions were evaluated.

Ten adult sheep, that is after reaching skeleton maturity , were used in total. Three millimeter diameter osteochondral lesions were made bilaterally at the level of the condylus and of the femoral trochlea. On one side, lesions were treated by perforations of the bottom and insertion of a membrane prepared as described in the example no. 8 (40% type II collagen - 60% type I collagen), fixed by mini-anchor with thread. As control, controlateral lesions were not treated. Animals were sacrificed at 3 and 9 months. In particular, the repair of an experimental type of complete traumatic lesion (osteochondral) was evaluated.

For microscopic analysis the samples, immediately after withdrawal, were fixed in 4% paraformaldehyde, 0.1 M phosphate buffer pH 7.4, decalcified in 4N formic acid and sodium citrate, embedded in paraffin and sectioned. Five-six micron thick sections were stained with hematoxylin-eosin, Alcian-PAS, Masson's Trichrome, Fullmer-Halmi.

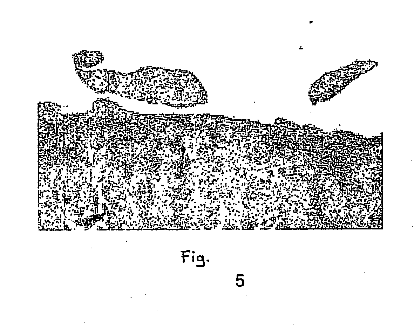

In the controls at 3 months, lesions of the joint surface appear empty or partially filled with fibrin clots (Figure 5). The lesion bed is later on invaded by hyghly vascularized granulation tissue with fusiform fibroblast-like cells. Aspects related to a proliferative and neochondrogenic activity of cartilaginous cells located at the lesion margins are never observed, and the lesion, in all cases, appears clearly delimited and morphologically distinct from the surrounding cartilage. Chondrocytes organized in clusters or islets, with aspects reminiscent of "brood capsules" in the arthritic process, are the expression of a reactive response to damage.

In the subsequent phases, the repair tissue coats the subchondral bone lamina and covers the lesion bed, like a cloth. The joint cartilage around the lesion shows early degenerative phenomena: disappearance of the columnar arrangement of the cellular component; notches from the more superficial layers deepen to the point to determine delamination and fibrillation of superficial and intermediate layers (Figure 6); alteration of the tintorial characteristics of the matrix; chondrocyte pycnosis, up to the appearance of more or less wide surface ulcerations; also the synovial membrane takes part in the pathological process triggered by the lesion, with congestion in the early phases, and later with formation of villi and of a thin "cloth" covering the altered cartilaginous surface.

In sheep treated with the membranes according to the invention, and described in example 8, after 3 months the lesion appears colonized by cells with some characteristics of the cartilaginous lineage. These cells are apparently viable, they are organized in clusters and are separated by a low abundant matrix that is intensely basophilic and poorly alcianophilic, indicating the presence of a matrix whose glycosaminoglycan content is still low.

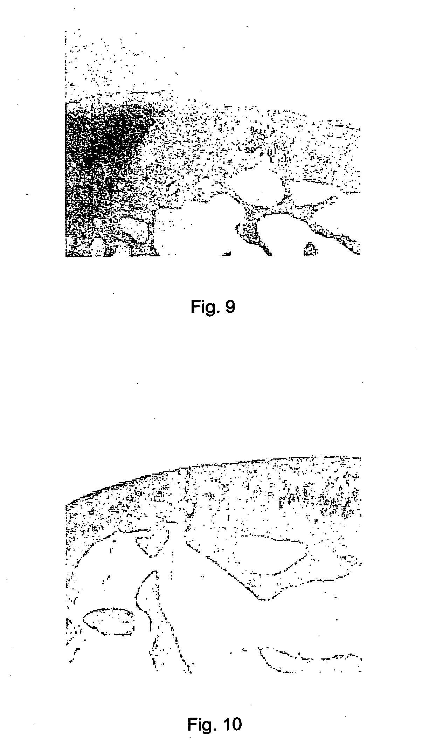

At 9 months, the lesion is filled by a repair tissue that is well anchored to the underlying bone and is composed by cartilaginous cells and by a hyaline cartilaginous matrix (Figures 7-8). It is possible to observe a tangential arrangement of the most superficial cells and, in the deepest portion of the repair tissue, a normal osteocartilaginous junction with formation of a *tide-mark* (see figures 9-10). The repair tissue is directly connected to the neighbouring joint cartilage, to restore the continuity of the articular surface (see figures 9 -10).

Histological evidence of cell-mediated immune reactions was not observed in any of the preparations.

Results of the application of the multimicrolamellar membrane, compared to a control lesion, can be seen in figures 6 and 7, which respectively relate to joint repair without membrane or in presence of a I/II membrane, after the same time period (9 months).

The relation between the newly formed cartilage and the surrounding tissues, at 9 months from application of the I/II membrane, are also evident in figure 8.
1) The application of multimicrolamellar membranes permeable to solutes but not to cells has a few primary effects:prevents the dispersion of osteochondral progenitor cells (mesenchymal, multipotent) from the bottom of the lesion to the articular environment, with marked reduction of the number of cells useful for repair;
2) favours the exchange of solutes required for feeding and proliferation of repair cells contained in the fibrin clot;
3) facilitates the integration of the repair tissue with the host tissue, contrasting the anti-adhesive effect observed by some authors that has been attributed to the synovial fluid rapidly "lacquering" the damaged surface.

The experiment shows that osteochondral lesions are repaired by filling the defect with a high number of osteochondral progenitor cells (mesenchymal, that is multipotent of stem type) coming from perforations of the subchondral bone. In addition to providing protection inside the lesion, the implant of collagen-based membranes according to the invention provides a three-dimensional support that is biologically suitable for invasion, proliferation and chondroblast differentiation of the above mentioned cells.

### Example 17. Differentiation and maintenance of the mesenchymal stromal cell phenotype

It was shown *in vitro* that chondrocytes proliferate and maintain their phenotype for a long time when they are cultured on multimicrolamellar membranes composed of type I and II collagen, and that pluripotent mesenchymal stromal cells are able to proliferate and differentiate as chondrocytes when they are cultured after appropriate separation on the same *scaffolds.*

Chondrocytes and adult mesenchymal stromal cells from bone marrow (MSC) were cultured on multimicrolamellar membranes made of type I collagen and of type II/I collagen in a ratio 40/60 (described above in example n.6 and n.8, respectively).

Cell proliferation was assessed by the MTT test, while differentiation was assessed by histochemical and immunohistochemical tests. Adult chondrocytes proliferated to a larger extent on the type II/I collagen membrane. MSC on type I collagen multimicrolamellar membrane proliferated and differentiated into cells that stained weakly with Safranin-O and produced type I collagen and chondroitin sulphate. Instead, on the type II/I collagen multimicrolamellar membrane, MSC differentiated in chondroblasts showing a clear immune reaction for the S-100 protein, chondroitin sulphate and type II collagen, which are typical reactions of cartilaginous cells (as described in : Grigolo B. et al., Biomaterials, 22, 2417, 2001).

**Example 18. Cartilage regeneration test *in vivo* upon implant of membranes obtained from collagen gels of the known art (comparative data)** An *in vivo* study, similar to that described in example 16, was carried out in rabbits, using membrane 40046 (EP1307247). As it is clear when comparing the section shown in figure 9, which relates to a control lesion at 12 months, with the section shown in figure 10, where the lesion was treated directly with the membrane known in the art, a tissue-repairing activity is observed at 12 months that is accompanied by formation of fibrocartilaginous tissue in both lesions. In fact, both figure 9 and figure 10 show the presence of the surgical lesion, and the repair tissue has the characteristics of fibrous tissue (Safranin O staining positive).

## Claims

1. Collagen membrane composed of at least 2 layers, wherein each layer is composed of a single type of collagen or a mixture of different types of collagens, **characterized by** a multimicrolamellar structure under scanning electron microscope.

2. Membrane according to claim 1 wherein at least one layer is type I collagen.

3. Membrane according to claims 1-2 wherein at least one layer is type II collagen.

4. Membrane according to claims 2-3 wherein at least one layer is a mixture of type I and type II collagen.

5. Membrane according to claim 4 wherein at least two layers are a mixture of type I and type II collagen.

6. Membrane according to claims 4-5 wherein said mixture contains type II collagen in a proportion comprised between 10-70% of the total collagen.

7. Membrane according to claim 6 wherein type II collagen is in a proportion comprised between 20-60%.

8. Membrane according to claim 7 wherein type II collagen is in a proportion comprised between 30-50%.

9. Membrane according to claim 5 having an imbibition capacity of at least 100% of its dry volume.

10. A process for the preparation of membranes from collagen gels according to claims 1-9, comprising the following steps:
a) electrostatic loading of a tray by its interposition in a vertical electrostatic field of at least 100 kv/m,
b) pouring a collagen gel in an electrostatically loaded tray and further interposition of the gel-containing tray in an electrostatic field of at least 100 kv/m with vertical orientation;
c) exsiccation of the collagen gel layer up to a residual water content of at least 30%,
d) pouring of at least one additional collagen gel layer;

11. The process according to claim 10 wherein, prior to electrostatic loading, trays are leant against a dispersion surface or otherwise electrostatically discharged.

12. The process according to claim 10 wherein the tray is electrostatically charged prior to pouring each subsequent layer of collagen gel.

13. The process according to claims 10-12 wherein the electrostatic field has a power comprised between 100-220 kv/m.

14. The process according to claims 10-13, wherein the tray is kept in the electrostatic field for at least 2'.

15. The process according to claims 10-14 wherein each collagen gel is flattened by use of a spatula.

16. The process according to claims 10-15 wherein in addition the following conditions take place, even independently one from the other:
a) the tray is of polymeric material;
b) the electrostatic field with vertical orientation is generated between two flat and parallel metal foils connected to a high voltage generator;
c) said electrostatic field has an intensity comprised between 150 and 200 kv/m, or more preferably between 160 and 180 kv/m;
d) the time of interposition of the tray in the electrostatic field is at least 5';
e) the partial exsiccation of the collagen gel takes place at a constant temperature comprised between 10 and 40°C, preferably between 20 and 30°C, even more preferably between 22-28°C, or even more preferably between 24-26°C, in an aerated incubator without turbulence, up to a residual water content comprised between 70% and 30%.

17. The process according to claims 10-15 wherein the step of exiccation of the last collagen gel layer is carried out at constant temperature and in absence of ventilation.

18. The process according to claims 10-17 wherein the exsiccation takes place up to a reduction of the collagen gel thickness comprised between 30-70% compared to the thickness of the volume that was initially poured, preferably 40-60%, even more preferably about 50%.

19. Process according to claims 10-17, wherein said tray is of polymeric material: polystyrene, polyethylene terephthalate (PET), polytetrafluoroethylene (teflon), polyvinyl chloride (pvc) etc

20. Process according to claim 19 wherein the tray is made of polystyrene.

21. Process according to claim 19 wherein the thickness of the poured collagen gel layer is from 1.5 to 20 mm.

22. Process according to claims 10-21 wherein the collagen gel consists of type I, type II and/or type III collagen, or is a mixture of one or more types of collagen.

23. Process according to claim 22 wherein said mixture of one or more types of collagen is a mixture of type I collagen and type II collagen.

24. Process according to claim 23 wherein said mixture comprises type II collagen in a proportion comprised from 0.2 to 0.6% (w/v).

25. Process according to claim 24 wherein type II collagen is in a proportion comprised from 0.3 to 0.5% (w/v).

26. Process according to claims 10-25 wherein the collagen layer that is poured consists of a collagen gel mixture comprising type II collagen gels.

27. Process according to claim 26 wherein the type II collagen gel is obtained by treating a tissue, source of type II collagen, with NaOH according to a weight ratio between grams of starting tissue and grams of NaOH not higher than 3.5, preferably from 1.8 to 2.8.

28. Process according to claims 10-27 wherein the poured collagen gel comprises type I collagen gel.

29. Process according to claim 28 wherein the type I collagen gel is obtained by treating a tissue, source of type I collagen, with NaOH according to a weight ratio between grams of starting tissue and grams of NaOH from 0.05 to 0.5, preferably from 0.1 to 0.3, even more preferably from 0.12 to 0.18, or is 0.15.

30. A multimicrolamellar membrane obtainable by the process according to claims 10-29.

31. Membrane according to claims 1-9 and 30 having a collagen content from 6 to 60 mg/cm², preferably from 9 to 30 mg/cm² and preferably from 15 to 25 mg/cm².

32. Membrane according to claims 1-9 and 30-31 having a yielding point value from 0.005 to 50 Mpa.

33. Membrane according to claim 32 wherein the yielding point value is comprised from 0.01 to 0.1 or from 0.5 to 50, more preferably from 2.5 to 15.

34. Membrane according to claims 1-9 and 30-32 having a Young modulus comprised from 0.001 to 100 Mpa.

35. Membrane according to claim 34 wherein the Young modulus is comprised from 0.01 to 0.1 Mpa or alternately from 0.5 to 60, more preferably comprised from 5 to 40.

36. Membrane according to claims 1-9 and 30-35 for reconstruction of osteo-cartilaginous tissue.

37. Membrane according to claims 1-9 and 30-35 for reconstruction of tendons, bones, joints.

38. Membrane according to claims 1-9 and 30-35 as support for non embryonic stem cells.

39. Membrane according to claim 38 as inducer of non embryonic stem cell differentiation.

40. Membranes according to claims 1-9 and 30-39 for osteochondral progenitor cellularization and subsequent cell differentiation *in situ.*

41. Use of membranes according to claims 1-9 and 30-40 as support for *in vitro* colonization by non embryonic stem cells.

## Patentansprüche

1. Kollagenmembran, welche sich aus mindestens zwei Schichten zusammensetzt, wobei jede Schicht aus einem einzigen Kollagentyp oder aus einer Mischung von verschiedenen Kollagentypen besteht, und welche **dadurch gekennzeichnet ist, dass** sie unter dem Scan-Elektronenmikroskop eine mikrolamellare Struktur aufweist.

2. Membran gemäß Anspruch 1, wobei mindestens eine Schicht aus Kollagen Typ I besteht

3. Membran gemäß Ansprüchen 1-2, wobei mindesten eine Schicht aus Kollagen Typ II besteht.

4. Membran gemäß Ansprüchen 2-3, wobei mindestens eine Schicht aus einer Mischung von Kollagen Typ I und Kollagen Typ II besteht.

5. Membran gemäß Anspruch 4, wobei mindestens zwei Schichten aus einer Mischung von Kollagen Typ I und Kollagen Typ II bestehen.

6. Membran gemäß Ansprüchen 4-5, wobei genannte Mischung Kollagen Typ II in einem Verhältnis enthält, welches zwischen 10-70 % des Gesamtkollagens umfasst.

7. Membran gemäß Anspruch 6, wobei Kollagen Typ II in einem Verhältnis enthalten ist, welches zwischen 20-60 % umfasst.

8. Membran gemäß Anspruch 7, wobei Kollagen Typ II in einem Verhältnis enthalten ist, welche zwischen 30-50 % umfasst.

9. Membran gemäß Anspruch 5, welche eine Aufnahmekapazität von mindestens 100 % ihres Trockenvolumens aufweist.

10. Verfahren für die Herstellung von Membranen aus Kollagengelen gemäß Ansprüchen 1-9, welches die folgenden Schritte umfasst:
a) elektrostatische Ladung eines Tabletts, indem das Tablett in ein vertikales elektrisches Feld von mindestens 100 kv/m eingebracht wird;
b) Gießen eines Kollagengels in ein elektrostatisch geladenes Tablett und weiteres Einbringen des Gel enthaltenden Tabletts in ein elektrostatisches Feld von mindestens 100 kv/m mit vertikaler Ausrichtung;
c) Trocknen der Kollagengelschicht bis zu einem Restwassergehalt von mindestens 30 %;
d) Gießen von mindestens einer zusätzlichen Kollagengelschicht;

11. Verfahren gemäß Anspruch 10, wobei Tabletts vor der elektrostatischen Ladung an eine Dispersionsfläche angelehnt werden oder auf andere Weise elektrostatisch entladen werden.

12. Verfahren gemäß Anspruch 10, wobei das Tablett elektrostatisch geladen wird, bevor eine jeweils weitere Schicht an Kollagengel eingegossen wird.

13. Verfahren gemäß Ansprüchen 10-12, wobei das elektrostatische Feld eine Stärke zwischen 100-220 kv/m umfasst.

14. Verfahren gemäß Ansprüchen 10-13, wobei das Tablett für mindestens 2'in dem elektrostatischen Feld verbleibt.

15. Verfahren gemäß Ansprüchen 10-14, wobei jedes einzelne Kollagengel unter Verwendung eines Spatels flach ausgestrichen wird.

16. Verfahren gemäß Ansprüchen 10-15, wobei zusätzlich die folgenden Bedingungen, auch unabhängig voneinander bestehen:
a.) Das Tablett besteht aus Polymermaterial;
b.) Das elektrostatische Feld mit vertikaler Ausrichtung wird zwischen zwei flachen und parallel angeordneten Metallfolien ausgerichtet, welche mit einem Starkstromgenerator verbunden sind;
c.) Genanntes elektrostatisches Feld weist eine Stärke auf, die zwischen 150 und 200 kv/m oder vorzugsweise zwischen 160 und 180 kv/m umfasst;
d.) Die Zeit, in der das Tablett in dem elektrostatischen Feld verbleibt, beträgt mindestens 5';
e.) Die teilweise Trocknung des Kollagengels findet in einem konstanten Temperaturbereich in einem belüfteten Inkubator ohne Turbulenzen statt, welcher zwischen 10 und 40 °C, vorzugsweise zwischen 20 und 30 °C und idealerweise zwischen 22-28 °C beträgt bis ein Restwassergehalt erreicht ist, der einen Bereich zwischen 70 %und 30 % umfasst.

17. Verfahren gemäß Ansprüchen 10-15, wobei der Schritt der Trocknung der letzten Kollagengelschicht bei konstanter Temperatur und ohne Ventilation durchgeführt wird

18. Verfahren gemäß Ansprüchen 10-17, wobei der Schritt der Trocknung bis zu einer Reduktion der Kollagengeldicke auf einen Bereich zwischen 30-70 %, vorzugsweise 40 -60 % und idealerweise etwa 50 % im Vergleich zu der Dicke des Volumen, welches zu Beginn eingegossen wurde stattfindet

19. Verfahren gemäß Ansprüchen 10-17, wobei genanntes Tablett aus Polymermaterial besteht: Polystyrol, Polyethylenterephtalat (PET), Polytetrafluorethylen (Teflon), Polyvinylchlorid (PVC) usw.

20. Verfahren gemäß Anspruch 19, wobei das Tablett aus Polystyrol besteht.

21. Verfahren gemäß Anspruch 19, wobei die Dicke der gegossenen Kollagengelschicht von 1,5 bis 20 mm beträgt.

22. Verfahren gemäß Ansprüchen 10-21, wobei das Kollagengel aus Kollagen von Typ I, Typ II und/oder von Typ III besteht oder es sich dabei um eine Mischung aus einem Kollagentyp oder mehreren Kollagentypen handelt.

23. Verfahren gemäß Anspruch 22, wobei genannte Mischung aus einem Kollagentyp oder aus mehreren Kollagentypen eine Mischung aus Kollagentyp I und Kollagentyp II ist.

24. Verfahren gemäß Anspruch 23, welches genannte Mischung Kollagen von Typ II in einem Verhältnis umfasst, welches von 0,2 bis 0,6 % (Gew.-Vol %) beträgt.

25. Verfahren gemäß Anspruch 24, welches Kollagen von Typ II in einem Verhältnis von 0,3 bis 0,5 % (Gew.-Vol.%) umfasst.

26. Verfahren gemäß Ansprüchen 10-25, wobei die Kollagenschicht, die eingegossen wird, aus einer Kollagengelmischung besteht, welche Kollagengele vom Typ II enthält.

27. Verfahren gemäß Anspruch 26, wobei Kollagengel vom Typ II erhalten wird, indem ein Gewebe, welches die Bezugsquelle für Kollagentyp II ist, mit NaOH behandelt wird gemäß einem Gewichtsverhältnis von Gramm Ausgangsgewebe zu Gramm NaOH, welches nicht höher als 3,5, vorzugsweise im Bereich von 1,8 bis 2,8 liegt.

28. Verfahren gemäß Ansprüchen 10-27, wobei das gegossene Kollagengel Kollagengel vom Typ I umfasst.

29. Verfahren gemäß Anspruch 28, wobei das Kollagengel vom Typ I erhalten wird, indem ein Gewebe, welches die Bezugsquelle für Kollagentyp I ist, gemäß einem Gewichtsverhältnis von Gramm Ausgangsgewebe zu Gramm NaOH, welches im Bereich von 0,05 bis 0,5, vorzugsweise von 0,1 bis 0,3 und idealerweise von 0,12 bis 0,18 oder 0,15 beträgt.

30. Multimikrolamellare Membran, welche durch das Verfahren gemäß Ansprüchen 10-29 erhalten wird.

31. Membran gemäß Ansprüchen 1-9 und 30, welche einen Kollagengehalt von 6 bis 60 mg/cm², vorzugsweise von 9 bis 30 mg/cm² und idealerweise von 15 bis 25 mg/cm² aufweist.

32. Membran gemäß Ansprüchen 1-9 und 30-31, welche einen Ertagspunktwert von 0,005 bis 50 Mpa aufweist.

33. Membran gemäß Anspruch 32, wobei der Ertragspunktwert einen Bereich von 0,01 bis 0,1 oder von 0,5 bis 50 und bevorzugter von 2,5 bis 15 umfasst.

34. Membran gemäß Ansprüchen 1-9 und 30-32, welche ein Young-Modul im Bereich von 0,001 bis 100 Mpa umfasst.

35. Membran gemäß Anspruch 34, wobei das Young-Modul einen Bereich von 0,01 bis 0,1 Mpa oder alternativ von 0,5 bis 60, bevorzugter von 5 bis 40 umfasst.

36. Membran gemäß Ansprüchen 1-9 und 30-35 für die Rekonstruktion von osteocartilaginärem Gewebe.

37. Membran gemäß Ansprüchen 1-9 und 30-35 für die Rekonstruktion von Sehnen, Knochen und Gelenken.

38. Membran gemäß Ansprüchen 1-9 und 30-35 als Träger von embryonalen Stammzellen.

39. Membran gemäß Anspruch 38 als Stimulans für nicht-embryonale Stammzelldifferenzierung.

40. Membranen gemäß Ansprüchen 1-9 und 30-39 für die osteochrondrale Progenitorzellbildung und nachfolgende Zelldifferenzierung *in situ.*

41. Verwenden der Membranen gemäß Ansprüchen 1-9 und 30-40 als Träger für die Kolonisation durch nicht-embryonale Stammzellen *in vitro.*

## Revendications

1. Membrane de collagène composée d'au moins 2 couches, où chaque couche se compose d'un seul type de collagène ou d'un mélange de différents types de collagène, **caractérisée par** une structure multimicrolamellaire sous microscope électronique au balayage.

2. Membrane selon la revendication 1 où au moins une couche est du collagène type I.

3. Membrane selon les revendications 1-2 où au moins une couche est du collagène type II.

4. Membrane selon les revendications 2-3 où au moins une couche est un mélange de collagène type I et type II.

5. Membrane selon la revendication 4 où au moins deux couches sont un mélange de collagène type I et type II.

6. Membrane selon les revendications 4-5 où ledit mélange contient du collagène type II à une proportion comprise entre 10 et 70% du collagène total.

7. Membrane selon la revendication 6 où le collagène type II est à une proportion comprise entre 20 et 60%.

8. Membrane selon la revendication 7 où le collagène type II est à une proportion comprise entre 30 et 50%.

9. Membrane selon la revendication 5 ayant une capacité d'imbibition d'au moins 100% de son volume sec.

10. Procédé pour la préparation de membranes à partir de gels de collagène selon les revendications 1-9, comprenant les étapes suivantes:
a) chargement électrostatiquement d'un plateau par son interposition dans un champ électrostatique vertical d'au moins 100 kv/m,
b) versement d'un gel de collagène dans un plateau électrostatiquement chargé et autre interposition du plateau contenant le gel dans un champ électrostatique d'au moins 100 kv/m avec orientation verticale;
c) dessèchement la couche de gel de collagène jusqu'à une teneur en eau résiduelle d'au moins 30%.
d) versement au moins une couche additionnelle de gel de collagène.

11. Procédé selon la revendication 10 où, avant charge électrostatique, les plateaux sont appuyés contre une surface de dispersion ou autrement électrostatiquement déchargés.

12. Procédé selon la revendication 10 où le plateau est électrostatiquement chargé avant de verser chaque couche subséquente du gel de collagène.

13. Procédé selon les revendications 10-12 où le champ électrostatique a une puissance comprise entre 100 et 220 kv/m.

14. Procédé selon les revendications 10-13 où le plateau est maintenu dans le champ électrostatique pendant au moins 2'.

15. Procédé selon les revendications 10-14 où chaque gel de collagène est aplati par l'utilisation d'une spatule.

16. Procédé selon les revendications 10-15 où, de plus, les conditions suivantes ont lieu, même indépendamment les unes des autres:
a) le plateau est en une matière polymérique;
b) le champ électrostatique avec orientation verticale est généré entre deux feuilles plates et parallèles de métal qui sont connectées à un générateur de haute tension;
c) ledit champ électrostatique a une intensité comprise entre 150 et 200 kv/m, ou mieux entre 160 et 180 kv/m;
d) le temps de l'interposition du plateau dans le champ électrostatique est d'au moins 5';
e) le dessèchement partiel du gel de collagène a lieu à une température constante comprise entre 10 et 40°C, de préférence entre 20 et 30°C, encore mieux entre 22 et 28°C, ou encore mieux entre 24 et 26°C, dans un incubateur aéré sans turbulence, jusqu'à une teneur en eau résiduelle comprises entre 70 et 30%.

17. Procédé selon les revendications 10-15 où l'étape de dessèchement de la dernière couche de gel de collagène est effectuée à une température constante et en l'absence de ventilation.

18. Procédé selon les revendications 10-17 où le dessèchement a lieu jusqu'à une réduction de l'épaisseur du gel de collagène comprise entre 30 et 70% en comparaison avec l'épaisseur du volume qui a été initialement versé, de préférence 40-60% et encore mieux environ 50%.

19. Procédé selon les revendications 10-17, où le plateau est en une matière polymérique: polystyrène, polyéthylène térephtalate (PET), polytétrafluoroéthylène (téflon), chlorure de polyvinyle (pvc) etc.

20. Procédé selon la revendication 19 où le plateau est fait de polystyrène.

21. Procédé selon la revendication 19 où l'épaisseur de la couche de gel de collagène versé est de 1,5 à 50 mm.

22. Procédé selon les revendications 10-21 où le gel de collagène consiste en collagène type I, type II et/ou type III, ou est un mélange d'un ou plusieurs types de collagène.

23. Procédé selon la revendication 22 où ledit mélange d'un ou plusieurs types de collagène est un mélange de collagène type I et collagène type II.

24. Procédé selon la revendication 23 où ledit mélange comprend du collagène type II à une proportion comprise entre 0,2 et 0,6% (p/v).

25. Procédé selon la revendication 24 où le collagène type II est à une porportion comprise entre 0,3 et 0,5% (p/V) .

26. Procédé selon la revendication 10-25 où la couche de collagène qui est versée consiste en un mélange de gel de collagène comprenant des gels de collagène type II.

27. Procédé selon la revendication 26 où le gel de collagène type II est obtenu en traitant un tissu, source de collagène type II, avec NaOH selon un rapport en poids entre les grammes du tissu de départ et les grammes de NaOH qui ne dépassent pas 3,5, de préférence de 1,8 à 2,8.

28. Procédé selon les revendications 10-27 où le gel de collagène versé comprend du gel de collagène type I.

29. Procédé selon la revendication 28 où le gel de collagène type I est obtenu par traitement d'un tissu, source de collagène type I avec NaOH selon un rapport en poids entre les grammes du tissu de départ et les grammes de NaOH de 0,05 à 0,5, de préférence de 0,1 à 0,3 et encore mieux de 0,12 à 0,18, ou est de 0,15.

30. Membrane multimicrolamellaire pouvant être obtenue par le procédé selon les revendications 10-29.

31. Membrane selon les revendications 1-9 et 30 ayant une teneur en collagène de 6 à 60 mg/cm², de préférence de 9 à 30 mg/cm² et de préférence de 15 à 25 mg/cm².

32. Membrane selon les revendications 1-9 et 30-31 ayant une valeur de limite élastique de 0,005 à 50 Mpa.

33. Membrane selon la revendication 32 où la valeur de limite élastique est comprise entre 0,01 et 0,1 ou entre 0,5 et 50 et mieux entre 2,5 et 15.

34. Membrane selon les revendications 1-9 et 30-32 ayant module de Young compris entre 0,01 et 100 Mpa.

35. Membrane selon la revendication 34 où le module de Young est compris entre 0,01 et 0,1 Mpa ou alternativement entre 0,5 et 60, mieux il est compris entre 5 et 40.

36. Membrane selon les revendications 1-9 et 30-35 pour une reconstruction d'un tissu ostéo-cartilagineux.

37. Membrane selon les revendications 1-9 et 30-35 pour une reconstruction de tendons, d'os, ou d'articulations.

38. Membrane selon les revendications 1-9 et 30-35 comme support pour des cellules souches non embryonnaires.

39. Membrane selon la revendication 38 comme inducteur de la différenciation des cellules souches non embryonnaires.

40. Membrane selon les revendications 1-9 et 30-39 pour la cellularisation progénétrice ostéochondrale et la différenciation cellulaire subséquent *in situ.*

41. Utilisation de membrane selon les revendications 1-9 et 30-40 comme support pour la colonisation *in vitro* par des cellules souches non embryonnaires.
